# FASCICULE DE BREVET EUROPEEN

(11) **EP 1 201 650 B1**
(45) Date de publication et mention de la délivrance du brevet: **22.11.2006**
(21) Numéro de dépôt: 01129670.4
(22) Date de dépôt: 30.12.1997
(51) Int. Cl.: C07C 317/44, C07C 255/17, C07C 255/65, C07C 255/27, C07C 255/05, C07C 255/35, C08F 220/44, C07C 255/31, C08G 65/48, C08G 73/06, C08G 77/44, C08G 73/02, C07F 17/02, C07F 7/18, C07C 311/02, C09K 3/00, H01M 6/16, H01M 10/40, C07B 41/00, C08F 4/00, C08J 3/24

(54) **Composés ioniques dérivés du malonitrile, et leurs utilisations**
Ionische Malonsäuredinitril Derivate und deren Verwendung
Ionic malonitril derivatives and their uses

(30) Priorité: 30.12.1996 CA 2194127; 05.03.1997 CA 2199231
(43) Date de publication de la demande: 02.05.2002
(62) Demande divisionnaire de: 97403189.0
(73) Titulaire: CENTRE NATIONAL DE LA RECHERCHE SCIENTIFIQUE (CNRS), 75016 Paris (FR); HYDRO-QUEBEC, Montréal, Québec H2X 3P3 (CA)
(72) Inventeur: Armand, Michel, Montreal, Quebec H3T 1N2 (CA); Gauthier, Michel, La Prairie, Quebec, J5R 1E6 (CA); Choquette, Yves, Sainte-Julie, Quebec, J3E 1P4 (CA); Michot, Christophe, 38000 Grenoble (FR)
(74) Mandataire: Sueur, Yvette

(56) Documents cités:
- WO-A-93/09092
- DD-A- 118 433
- US-A- 4 966 954
- US-A- 5 232 940
- A. DORNOW ET AL.: "Über Umsetzungen von alpha-Ketonitrilen, V. Über die Verwendung von alpha-Ketonitrilen zur C-Alkylierung" CHEMISCHE BERICHTE, vol. 91, no. 9, 1958, pages 1824-29, XP002061822 WEINHEIM, DE
- H. HIROTA ET AL.: "facile carbon-carbon bond heterolysis. SN1-E1 Reactions of t-cumyl and t-butyl substituted tricyanomethanes" CHEMISTRY LETTERS, no. 5, mai 1990 (1990-05), pages 803-6, XP002061823 TOKYO, JP
- H.W. ROESKY ET AL.: "Synthese und Struktur des Trifluoracetyldicyanomethanids" ZEITSCHRIFT FÜR NATURFORSCHUNG, TEIL B, ANORGANISCHE CHEMIE, ORGANISCHE CHEMIE, vol. 40b, no. 7, juillet 1985 (1985-07), pages 883-5, XP002061824 TÜBINGEN, DE
- J.A. ELVIDGE ET AL.: "Preparation of some highly polarised ethenes by the addition of amines to suitable carbonitriles" JOURNAL OF THE CHEMICAL SOCIETY, PERKIN TRANSACTIONS I, no. 8, 1983, pages 1741-4, XP002061825 LONDON, GB
- E. OTT ET AL.: "Zur Kenntnis einfacher Cyanverbindungen, IV. Mitteil.: Über das Dibrom-malonitril und seine Umwandlung in Natrium-azidomalonitril und in ein dimolekulares Cyan-azid C2N8" CHEMISCHE BERICHTE, vol. 70, no. 8, 1937, pages 1829-34, XP002061826 WEINHEIM, DE
- J.-P. FLEURY ET AL.: "Carboacides polycyanés. II. Sulfonylmalodinitriles. Préparation, structure et propriétés" BULLETIN DE LA SOCI¹T¹ CHIMIQUE DE FRANCE, 1966, pages 1966-70, XP002061827 PARIS, FR
- B.K. FREED ET AL.: "(Alpha-perfluoroheptyl-beta,beta- dicyanovinyl)aminostyrenes: a novel class of perfluorocarbon-soluble dyes" JOURNAL OF FLUORINE CHEMISTRY, vol. 47, no. 2, mai 1990 (1990-05), pages 219-25, XP002061828 LAUSANNE, CH
- DATABASE CROSSFIRE BEILSTEIN [en ligne] Beilstein Informationssysteme GmbH, Frankfurt DE; XP002061829 & J. ORG. CHEM. USSR (ENGL. TRANSL.), vol. 27, no. 6.1, 1991, page 1042-8

## Description

La présente invention a pour objet des composés ioniques dérivés du malononitrile dans lesquels la charge anionique est délocalisée, et leurs utilisations.

Il est connu et particulièrement intéressant d'introduire des groupements ioniques dans les molécules ou les polymères organiques possédant des fonctions variées. Les forces coulombiennes correspondent, en effet, aux interactions les plus fortes disponibles au niveau moléculaire, et les groupements ioniques modifient de la manière la plus marquée les molécules auxquelles ils sont attachés. On peut citer les colorants qui sont rendus solubles dans l'eau à l'aide de fonctions sulfonates ou carboxylates.

Cependant les groupements de ce type -CO₂⁻ 1/mM^{m+} ou -SO₃⁻ 1/mM^{m+} ne sont pas dissociés, et ils n'induisent pas de solubilité dans les solvant autres que l'eau ou certains solvants protiques très polaires comme les alcools légers, ce qui restreint considérablement la portée de leur utilisation.

On connaît par ailleurs **(EP-A-0 290 511)** les sels des composés [R_{F}SO₂-N-SO₂R_{F}]⁻ 1/mM^{m+} dans lesquels R_{F} est un groupement perfluoré et M^{m+} un cation à la valence m+ qui sont solubles et dissociés dans les milieux aprotiques organiques ou les polymères solvatants. Il est cependant considéré que l'existence de deux groupements perfluoroalkylsulfonyles (en particulier l'existence d'atomes de fluor sur l'atome de carbone en α de chacun des groupements sulfonyles) qui exercent un pouvoir attracteur important sur les électrons de la charge anionique, est une condition nécessaire à l'obtention des propriétés de solubilité et dissociation. Pour exemple, le pKₐ de l'acide H[CF₃SO₂-N-SO₂CF₃] n'est que de 1,95, comparable à celui de l'acide non fluoré CH₃SO₃H (pKₐ = 0,3) et nettement inférieur à celui de l'acide perfluoré CF₃SO₃H (pKₐ < -9) du fait de la basicité de l'atome d'azote central.

On connaît en outre par WO93/09092 des composés répondant à la formule (R_{F}SO₂)₂CY⁻M⁺ et des matériaux à conduction ionique qui les contiennent. R_{F} représente un groupe perfluoroalkyle, M une espèce cationique minérale ou organique et Y peut être un groupe nitrile.

D'une manière surprenante, les inventeurs ont trouvé que les composés contenant des groupements ioniques -C(CN)₂⁻ avaient d'excellentes propriétés de solubilité et de dissociation, même lorsqu'ils ne contiennent pas de groupements perfluorés fortement électroattracteurs.

La présente invention a par conséquent pour but de fournir une famille de composés ioniques possédant une bonne solubilité et une bonne dissociation, sans qu'il soit nécessaire de faire appel à des modifications complexes de la molécule de départ. Les précurseurs des molécules de l'invention sont pour la plupart des produits industriels et/ou facilement accessibles. Il est à noter en outre que l'absence, ou tout au moins la diminution de la fraction perfluorée dans les composés de l'invention permet de réduire les coûts de production de composés et par conséquent le coût des applications qui en sont faites.

Un objet de la présente invention est un composé ionique dérivé du malononitrile comprenant une partie anionique associée à au moins une partie cationique M^{+m} en nombre suffisant pour assurer la neutralité électronique de l'ensemble, caractérisé en ce que la partie anionique répond à l'une des formules R_{D}-Y-C(C≡N)₂⁻ ou Z-C(C≡N)₂⁻ dans lesquelles :
- Z représente un radical électro-attracteur ayant un paramètre de Hammett au moins égal à celui d'un radical phényle, choisi parmi :
   j) -SCN, -N₃, FSO₂-, -CF₃, R'_{F}CH₂- (R'_{F} étant un radical perfluoré, de préférence CF₃-), les radicaux fluoroalkyloxy, fluoroalkylthioxy, fluoroalkényloxy, fluoroalkénylthioxy ;
   jj) les radicaux comprenant un ou plusieurs noyaux aromatiques contenant éventuellement au moins un atome d'azote, d'oxygène, de soufre ou de phosphore, les dits noyaux pouvant être éventuellement des noyaux condensés et/ou lesdits noyaux pouvant éventuellement porter au moins un substituant choisi parmi les halogènes, -CN, -NO₂, -SCN, -N₃, CF₂=CF-O-, les radicaux R_{F}- et R_{F}CH₂- dans lesquels R_{F} est un radical perfluoroalkyle ayant de 1 à 12 atomes de carbone, les groupes fluoroalkyloxy, les groupes fluoroalkylthioxy, les radicaux alkyles, alkényles, oxa-alkyles, oxaalkényles, azaalkyles, azaalkényles, thiaalkyles, thiaalkényles, les radicaux polymères, les radicaux possédant au moins un groupement ionophore cationique et/ou au moins un groupement ionophore anionique ;
- Y représente un groupement sulfonyle ou un groupement phosphonyle et :
- R_{D} est un radical choisi parmi :
   a) les radicaux alkyle ou alkényle, les radicaux aryle, arylalkyle, alkylaryle ou alkénylaryle, les radicaux alicycliques ou hétérocycliques, y compris les radicaux polycycliques ;
   b) les radicaux alkyle ou alkényle comprenant au moins un groupe fonctionnel éther, thioéther, amine, imine, amide, carboxyle, carbonyle, isocyanate, isothiocyanate, hydroxy ;
   c) les radicaux aryle, arylalkyle, arylalkényle, alkylaryle ou alkénylaryle, dans lesquels les noyaux aromatiques et/ou au moins un substituant du noyau comprennent des hétéroatomes tels que l'azote, l'oxygène, le soufre ;
   d) les radicaux comprenant des cycles aromatiques condensés qui comprennent éventuellement au moins un hétéroatome choisi parmi l'azote, l'oxygène, le soufre ;
   e) les radicaux halogénés ou perhalogénés alkyle, alkényle, aryle, arylalkyle, alkylaryle, lesdits radicaux comprenant éventuellement des groupes fonctionnels éther, thioéther, imine, amine, carboxyle, carbonyle ou hydroxy ;
   f) les radicaux R_{c}C(R')(R'')-O- dans lesquels R_{C} est un radical alkylperfluoré et R' et R" sont indépendamment l'un de l'autre un atome d'hydrogène ou un radical tel que défini en a), b), c) ou d) ci-dessus [par exemple CF₃CH₂O-, (CF₃)₃CO-, (CF₃)₂CHO-, CF₃CH(C₆H₅)O-, -CH₂(CF₂)₂CH₂-];
   g) les radicaux (R_{B})₂N-, dans lesquels les radicaux R_{B} identiques ou différents sont tels que définis en a), b), c), d) et e) ci-dessus, l'un des R_{B} pouvant être un atome d'hydrogène, ou bien les deux radicaux R_{B} forment ensemble un radical divalent qui forme un cycle avec N ;
   h) les radicaux polymères,
   i) les radicaux possédant un ou plusieurs groupements ionophores cationiques et/ou un ou plusieurs groupements ionophores anioniques ;
   et en ce que le cation est un cation onium choisi dans le groupe constitué par les cations R₃O⁺ (oxonium), NR₄⁺ (ammonium), RC(NR₂)₂⁺ (amidinium), C(NR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium). Dans un cation onium donné, les radicaux R peuvent être tous identiques. Mais un cation onium peut aussi comporter des radicaux R différents les uns des autres. Un radical R peut être un H ou bien il est choisi parmi les radicaux suivants :
- les radicaux alkyles, alkényles, oxa-alkyles, oxa-alkényles, aza-alkyles, aza-alkényles, thia-alkyles, thia-alkényles, aryles, arylalkyles, alkylaryles, alkénylaryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore ;
étant entendu que :
- les composés C₆H₅SO₂C(CN)₂NH₄, C₆H₅SO₂C(CN)₂C₆H₅CH₂-SC(NH₂)NH, C₆H₅SO₂C(CN)₂C₆H₅NH₃, m-NO₂C₆H₅SO₂C(CN)₂(EH₃NH, C₆H₅SO₂C(CN)₂C₅H₁₀NH et C₆H₅SO₂C(CN)₂C₆H₅NH sont exclus ;
- un substituant Z peut être un radical monovalent, une partie d'un radical ayant une valence égale à 2 portant deux groupements -C-(C≡N)₂' ou un segment d'un polymère ;
- un substituant R_{D} peut être un radical monovalent, une partie d'un radical ayant une valence supérieure à 2 portant plusieurs groupements -Y-C⁻(C≡N)₂' ou un segment d'un polymère ;
- "segment d'un polymère" signifie unité récurrente éthylène portant éventuellement un substituant alkylène ou un substituant oxyalkénylène, unité récurrente oxyalkylène portant éventuellement un groupe méthylène (dérivée d'un 2,3-epoxypropane), unité récurrente styrène portant éventuellement un substituant polyoxyéthylène, unité récurrente azaéthylène, unité récurrente 2-(triéthoxysilyl)éthyl-co-O-[2-(triméthoxysilyl)éthyl]-O'-méthylpolyéthylène glycol, unité récurrente bis[3-triméthoxysilyl)propyl]amino, unité récurrente méthyléthylsiloxane, unité récurrente acrylonitrile-co-4-styrényle, ou unité récurrente aniline portant un substituant fluoroalkyle ;
- plusieurs radicaux R peuvent former ensemble des cycles aliphatiques ou aromatiques englobant éventuellement le centre portant la charge cationique ;
- "groupement ionophore cationique" signifie un groupement iodonium, sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation M⁺ ;
- "groupement ionophore anionique" signifie une fonction carboxylate (-CO₂⁻), une fonction sulfonate (-SO₃⁻), une fonction sulfonimide (-SO₂NSO₂-) ou une fonction sulfonamide (-SO₂N-).

Lorsqu'un cation onium porte au moins deux radicaux R différents de H, ces radicaux peuvent former ensemble un cycle aromatique ou non, englobant éventuellement le centre portant la charge cationique.

Lorsque la partie cationique d'un composé de l'invention est un cation onium, il peut se présenter soit sous la forme d'un groupe cationique indépendant qui n'est lié à la partie cationique que par la liaison ionique entre la charge positive du cation et la charge négative de la partie anionique. Dans ce cas, la partie cationique peut faire partie d'une unité récurrente d'un polymère.

Un cation onium peut également faire partie du radical Z ou du radical R_{D} porté par le centre anionique. Dans ce cas, un composé de l'invention constitue un zwitterion. Lorsque le cation d'un composé de l'invention est un cation onium, il peut être choisi de telle sorte à introduire dans le composé des substituants permettant de conférer audit composé des propriétés spécifiques. Par exemple, le cation M⁺ peut être un hétérocycle cationique à caractère aromatique, comportant au moins un atome d'azote alkylé dans le cycle. A titre d'exemple, on peut citer un imidazolium, un triazolium, un pyridinium, un 4-diméthylamino-pyridinium, lesdits cations portant éventuellement un substituant sur les atomes de carbone du cycle. Parmi ces cations, ceux dont les sels ont un point de fusion inférieur à 150°C, plus particulièrement inférieur à 25°C.

Un composé de l'invention dans lequel le cation M est un groupement cationique possédant un groupement diazoïque -N=N-, -N=N⁺, un groupement sulfonium, un groupement iodonium, un groupement phosphonium ou un cation arène-ferrocénium substitué ou non, éventuellement incorporé dans une trame polymérique, est intéressant dans la mesure où il est activable par une source d'énergie actinique de longueur d'onde appropriée. Comme exemples particuliers de tels composés, on peut citer ceux dans lesquels le cation est un cation diaryliodonium, un cation dialkylaryliodonium, un cation triarylsulfonium, un cation trialkylaryle sulfonium, ou un cation phénacyl-dialkyl sulfonium substitué ou non. Les cations précités peuvent faire partie d'une chaîne polymère.

Le cation M d'un composé de l'invention peut être un cation organique incorporant un groupement 2,2'[Azobis(2-2'-imidazolinio-2-yl)propane]²⁺ ou 2,2'-Azobis(2-amidinio-propane)²⁺. Le composé de l'invention est alors particulièrement intéressant comme initiateur radicalaire, activable thermiquement et non volatil, soluble dans les solvants organiques polaires et dans les monomères et polymères solvatants aprotiques.

Une famille particulière de composés de l'invention est celle qui comprend un groupe R_{D}Y-. Les composés dans lesquels Y est -SO₂- sont spécialement préférés.

Le choix du substituant R_{D} permet d'ajuster les propriétés d'un composé de l'invention. Dans un mode de réalisation, R_{D} est choisi parmi les radicaux alkyles, alkényle, oxa-alkyle, oxa-alkényle, aza-alkyle, aza-alkényle, thia-alkyle ou thia-alkényle ayant de 1 à 24 atomes de carbone, ou parmi les radicaux aryle, arylalkyle, alkylaryle ou alkénylaryle ayant de 5 à 24 atomes de carbones.

Dans un autre mode de réalisation, R_{D} est choisi parmi les radicaux alkyles ou alkényles ayant de 1 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine ou un groupe carboxyle.

Dans un autre mode de réalisation, R_{D} est choisi parmi les radicaux aryle, arylalkyle, alkylaryle ou alkénylaryle, dans lesquels les noyaux aromatiques et/ou leurs substituants comprennent des hétéroatomes tels que l'azote, l'oxygène, le soufre.

Un substituant R_{D} peut être un radical polymère, par exemple un radical poly(oxyalkylène). Le composé de l'invention se présente alors sous la forme d'un polymère portant un groupe ionique -Y-C(CN)₂⁻, M⁺.

R_{D} peut être une unité récurrente d'un polymère, par exemple une unité oxyalkylène ou une unité styrène. Le composé de l'invention se présente alors sous la forme d'un polymère dans lequel une partie au moins des unités récurrentes portent un groupe latéral sur lequel est fixé un groupe ionique -Y-C(CN)₂⁻, M⁺. A titre d'exemple, on peut citer un poly(oxyalkylène) dans lequel au moins certaines unités oxyalkylène portent un substituant -Y-C(CN)₂⁻, M⁺, ou un polystyrène dans lequel au moins certaines unités styrène portent un substituant -Y-C(CN)₂⁻, M⁺.

Une catégorie particulière de composés selon l'invention comprend les composés dans lesquels le substituant R_{D} possède au moins un groupement ionophore anionique et /ou au moins un groupement ionophore cationique. Le groupement anionique peut par exemple être une fonction carboxylate (-CO₂⁻), une fonction sulfonate (-SO₃⁻), une fonction sulfonimide (-SO₂NSO₂-) ou une fonction sulfonamide (-SO₂N-). Le groupement ionophore peut par exemple être un groupement iodonium, sulfonium, oxonium, ammonium, amidinium, guanidinium, pyridinium, imidazolium, imidazolinium, triazolium, phosphonium ou carbonium. Le groupement ionophore cationique peut jouer totalement ou partiellement le rôle du cation M.

Lorsque R_{D} comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes ionophores sur les polymères portant la fonction réactive appropriée.

Un substituant R_{D} peut être un groupe mésomorphe ou un groupe chromophore ou un polymère conducteur électronique autodopé ou un alcoxysilane hydrolysable.

Un substituant R_{D} peut comporter un groupement susceptible de piéger les radicaux libres, par exemple un phénol encombré ou une quinone.

Un substituant R_{D} peut aussi comporter un dipôle dissociant, par exemple une fonction amide, une fonction sulfonamide ou une fonction nitrile.

Un substituant R_{D} peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imine aromatique.

Un substituant R_{D} peut aussi comporter un ligand complexant, ou un groupe optiquement actif.

Selon une autre variante, un composé selon l'invention comprend un substituant R_{D} qui représente un radical ayant une valence v supérieure à deux, comportant lui-même au moins un groupe -Y-C(CN)₂⁻, M⁺. Dans ce cas, les charges négatives présentes sur la partie anionique du composé de l'invention devront être compensées par le nombre approprié de cations ou de groupes ionophores cationiques M.

Lorsqu'un composé de la présente invention répond à la formule Z-C(CN)₂⁻, M⁺ dans laquelle Z est un groupement électroattracteur non lié à l'azote portant la charge négative par un groupe -SOₓ-, Z est avantageusement choisi dans le groupe constitué par -OCₙF₂ₙ₊₁, -OC₂F₄H, -SCₙF₂ₙ₊₁ et -SC₂F₄H, -OCF=CF₂, -SCF=CF₂, n étant un nombre entier de 1 à 8. Z peut également être un radical CₙF₂ₙ₊₁CH₂-, n étant un nombre entier de 1 à 8.

Les composés de la présente invention peuvent être obtenus par un procédé dans lequel on fait réagir un composé R_{D}-Y-L ou Z-L avec un composé [A-C(CN)₂]ⁿ⁻ ₘ nM'^{m+},
- Z et R_{D} étant tels que définis précédemment,
- M' étant H ou un cation tel que défini précédemment pour M,
- L représentant un groupe partant électronégatif tel qu'un halogène, un radical N-imidazoyle, un radical N-triazoyle, un composé donnant un ester activé (par exemple un succinimidyloxy, un benzotriazoloxy ou une O-acylurée), un groupement alcoxyde, un groupement R_{D}-Y-O- ou un groupement R_{D}-Y-S-, et
- A représentant un cation M^{m+}, un groupe trialkylsilyle, un groupe trialkyle germanyle, un groupe trialkylstannyle ou un groupe tertioalkyle, dans lesquels les substituants alkyles ont de 1 à 6 atomes de carbone.

A titre d'exemple, on peut citer la réaction d'un fluorure de fluorosulfonyle avec un di sel de malononitrile selon le schéma réactionnel suivant :

FSO₂-F + [NaC(CN)₂]⁻ Na⁺ ⇒ NaF + [FSO2-C(CN)₂]⁻ Na⁺.

L'utilisation d'un composé [A-C(CN)₂]ⁿ⁻ ₘ nM'^{m+} dans lequel A est un groupement tertioalkyle est avantageuse, car un tel groupement est précurseur de proton par formation de l'alcène correspondant. L'utilisation du groupe trialkylsilyle est spécialement intéressante lorsque le groupe partant est un fluor, du fait de la très grande stabilité de la liaison F-Si.

Lorsque l'on utilise un composé [A-C(CN)₂]ⁿ⁻ ₘ nM'^{m+} dans lequel A est le proton, il est avantageux d'effectuer la réaction en présence d'une base tertiaire ou d'une base encombrée T susceptible de former le sel L⁻(HT⁺) par combinaison avec le proton, afin de favoriser la formation du composé de l'invention. La base peut être choisie parmi les alkylamines (par exemple la triéthylamine, la di-isopropylamine, la quinuclidine), le 1,4-diazobicyclo[2,2,2]octane (DABCO) ; les pyridines (par exemple la pyridine, les alkylpyridines, les dialkylaminopyridines) ; les imidazoles (par exemple les N-alkylimidazoles, l'imidazo[1,1-a]pyridine) ; les amidines (par exemple le 1,5-diazabicyclo-[4,3,0]non-5-ène (DBN), le 1,8-diazabicyclo[5,4,0]undec-7-ène (DBU)) ; les guanidines (par exemple la tétraméthyl guanidine, la 1,3,4,7,8-hexahydro-1-méthyl-2H-pyrimido[1,2-a]pyrimidine (HPP)). On peut également utilisé un sel de métal alcalin du malononitrile comme base.

A titre d'exemple d'un tel procédé, on peut citer le procédé dans lequel on fait réagir un chlorure de carbonyle R_{D}COCl avec le malononitrile en présence de DABCO.

Un composé selon l'invention peut également être obtenu par couplage direct entre un sel du malononitrile et un acide carboxylique à l'aide d'un agent de couplage. Lorsque Z = CO, il est avantageux d'utiliser un composé RZX du type pseudo-halogénure préparé directement *in-situ* (X = RCO₂, SCO, PTO, BzO) à partir de RCOOH par action des agents de condensation utilisés dans la synthèse des peptides (déshydratants moléculaires). De tels agents sont décrits par exemple dans *Synthesis* p. 453 (1972) et dans *Ann. Rev. Biochem* 39, 841 (1970). Les composés de l'invention sont alors préparés à partir de RCOOH auquel est ajouté le déshydratant moléculaire,puis le composé (1/nM)[(NC)₂CH] en proportions stoechiométriques, dans un solvant polaire. De préférence, l'agent de condensation est choisi parmi les carbodiimides, par exemple le cyclohexyl carbodiimide ou le diisopropyl carbodiimide ; les carbonates et les oxalates de succinimidyle, de phtalimidyle, de 1-benzotriazolyle, de nitro-, de dinitro- ou de perhalo-phénols, de trifluoroéthyle, de trichloroéthyle ; le mélange PΦ₃-diéthylazodicarboxylate (DEAD) ou PΦ₃-dithiodipyridine ; le carbonyldiimidazole -(Im)₂CO ou le phénylphosphoro-diimidazole ΦPO(Im)₂ ; les acétals d'amides, par exemple, le diméthylformamide di-néopentyl acétal (CH₃)₂NCH[OCH₂C(CH₂)₂]₂ ; les 2-alcoxy-1-alcoxycarbonyl-1,2-dihydroquinoline ; les sels de O-benzotriazol-1-yl-N,N,N',N'-tétraméthyluronium ou de O-benzo triazol-1-yloxytrisdiméthylaminophosphonium ; les sultones aromatiques, par exemple, l'oxyde de 2,2-(6-nitronapht[1,8-cd]-1,2-oxathiole), le chloroformiate d'isobutyle, le diphé-nylphosphochloroiridate, le chlorophosphite d'éthylène, le pyrophosphite de diéthyléthylène ; le chlorure de bis(2-oxo-3-oxazolidinyl)phosphinyle ; les sels de 2-*ter*-butyl-5-méthyl isooxazolium (Woodward's reagent L).

Le cation d'un composé obtenu selon l'un ou l'autre des procédés décrits ci-dessus peut être remplacé par les procédés classiques d'échange de cation, soit par des précipitations ou des extractions sélectives, soit par l'utilisation de résines échangeuses d'ions.

En outre, le substituant R_{D} d'un composé de l'invention peut être modifié par les réactions connues. Par exemple, un substituant R_{D} qui comprend un groupe allyle peut être transformé par réaction avec un peroxyde pour obtenir un substituant R_{D} époxydé. Un groupe -NHR peut être transformé en groupe vinylester par réaction avec du tert-butoxyde de potassium et le vinylchloroformate. Les procédés pour réaliser ces modifications et d'autres sont à la portée de l'homme de métier.

Les composés ioniques de la présente invention comprennent au moins un groupement ionophore sur lequel sont fixés des substituants qui peuvent être très variés. Compte tenu du grand choix possible pour les substituants, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. Les composés de l'invention qui sont des polymères, de même que des composés polymères obtenus à partir de composés de l'invention ayant la propriété de se polymériser ou de se copolymériser, présentent les propriétés énumérées ci-dessus avec l'avantage d'avoir d'une charge anionique immobile.

Il a été observé que la forte dissociation des espèces ioniques des composés de l'invention se traduisait par une stabilisation des carbocations, en particulier ceux dans lesquels il existe une conjugaison avec l'oxygène ou l'azote et, d'une manière surprenante, par une forte activité de la forme protonée des composés l'invention sur certains monomères. La présente invention a donc également pour objet l'utilisation des composés ioniques comme photoinitiateurs sources d'acides de Brønsted catalyseurs de polymérisation ou de réticulation de monomères ou de prépolymères capables de réagir par voie cationique, ou comme catalyseurs pour la modification de polymères.

Le procédé de polymérisation ou de réticulation de monomères ou de prépolymères capables de réagir par voie cationique est caractérisé en ce que l'on utilise un composé de l'invention comme photoinitiateur source d'acide catalysant la réaction de polymérisation. Les composés selon l'invention dans lesquels le cation est un groupement possédant une liaison -N=N⁺, -N=N-, un groupement sulfonium, un groupement iodonium, ou un cation arène-ferrocénium substitué ou non, éventuellement incorporé dans une trame polymérique, sont particulièrement préférés.

Le choix du substituant R_{D} ou du substituant Z est effectué de manière à augmenter la solubilité dudit composé dans les solvants utilisés pour la réaction des monomères ou des prépolymères, et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donnera une solubilité dans les milieux polaires. Les radicaux incluant un groupement sulfoxyde, un groupement sulfone, un groupement oxyde de phosphine, un groupement phosphonate, obtenus respectivement par addition d'oxygène sur les atomes de soufre ou de phosphore, peuvent conférer au polymère obtenu des propriétés améliorées en ce qui concerne l'adhésion, la brillance, la résistance à l'oxydation ou aux UV. Les monomères et les prépolymères qui peuvent être polymérisés ou réticulés à l'aide des photoinitiateurs de la présente invention sont ceux qui peuvent subir une polymérisation cationique.

Les monomères et les prépolymères qui peuvent être polymérisés ou réticulés à l'aide des photoinitiateurs de la présente invention sont ceux qui peuvent subir une polymérisation cationique.

Parmi les monomères, on peut citer les monomères qui comportent une fonction éther cyclique, une fonction thioéther cyclique ou une fonction amine cyclique les composés vinyliques, (plus particulièrement les éthers vinyliques), les oxazolines, les lactones et les lactames.

Parmi les monomères du type éther ou thioéther cyclique, on peut citer l'oxyde d'éthylène, l'oxyde de propylène, l'oxétane, l'épichlorhydrine, le tétrahydrofurane, l'oxyde de styrène, l'oxyde de cyclohexène, l'oxyde de vinylcyclohexène, le glycidol, l'oxyde de butylène, l'oxyde d'octylène, les éthers et les esters de glycidyle (par exemple le méthacrylate ou l'acrylate de glycidyle, le phényl glycidyl éther, le diglycidyléther de bisphénol A ou ses dérivés fluorés), les acétals cycliques ayant de 4 à 15 atomes de carbone (par exemple le dioxolane, le 1,3-dioxane, le 1,3-dioxépane) et les spiro-bicyclo dioxolanes.

Parmi les composés vinyliques, les éthers vinyliques constituent une famille très importante de monomères sensibles en polymérisation cationique. A titre d'exemple, on peut citer l'éthyl vinyl éther, le propyl vinyl éther, l'isobutyl vinyl éther, l'octadécyl vinyl éther, l'éthylèneglycol monovinyl éther, le diéthylèneglycol divinyl éther, le butanediol monovinyl éther, le butanediol divinyl éther, l'hexanediol divinyl éther, l'éthylèneglycol butyl vinyl éther, le triéthylèneglycol méthyl vinyl éther, le cyclohexanediméthano monovinyl éther, le cyclohexanediméthanol divinyl éther, le 2-éthylhexyl vinyl éther, le poly-THF-divinyl éther ayant une masse comprise entre 150 et 5000, le diéthylèneglycol monovinyl éther, le triméthylolpropane trivinyl éther, l'aminopropyl vinyl éther, le 2-diéthylaminoéthyl vinyl éther.

Comme autres composés vinyliques, on peut citer à titre d'exemple les 1,1-dialkyléthylènes (par exemple l'isobutène), les monomères aromatiques vinyliques (par exemple le styrène, les α-alkylstyrènes, notamment l'α-méthylstyrène, le 4-vinylanisole, l'acénaphtène), les composés N-vinyliques (par exemple la N-vinylpyrolidone ou les N-vinyl sulfonamides).

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène, les siloxanes possédant des groupements latéraux du type époxycyclohexène-éthyle obtenus par hydrosilylation des copolymères de di alkyl, d'alkylaryle ou de diaryl siloxane avec le méthyl hydrogénosiloxane en présence d'oxyde de vinylcyclohexène, les produits de condensation du type sol-gel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention consiste à mélanger au moins un monomère ou prépolymère capable de polymériser par voie cationique et au moins un composé ionique de l'invention, et à soumettre le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 µm. La durée de la réaction dépend de l'épaisseur de l'échantillon et de la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de photoinitiateur utilisé est comprise entre 0,01 et 15 % en poids par rapport au poids de monomère ou de prépolymère, de préférence entre 0,1 et 5 % en poids.

Un composé ionique de la présente invention peut être utilisé comme photoinitiateur en l'absence de solvant, notamment lorsque l'on souhaite polymériser des monomères liquides dans lesquels le composé ionique utilisé comme photoinitiateur est soluble ou aisément dispersable. Cette forme d'utilisation est particulièrement intéressante, car elle permet de supprimer les problèmes liés aux solvants (toxicité, inflammabilité).

Un composé ionique de la présente invention peut également être utilisé en tant que photoinitiateur sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. Ces solvants serviront simplement à diluer les produits à polymériser ou à réticuler (pour les rendre moins visqueux, surtout lorsqu'il s'agit d'un prépolymère). Ils seront éliminés après la polymérisation ou la réticulation par séchage. On peut également citer les solvants non volatils. Un solvant non volatil sert également à diluer les produits que l'on veut polymériser ou réticuler, et à dissoudre le composé ionique de l'invention utilisé comme photoinitiateur, mais il restera dans le matériau formé et il agit ainsi comme plastifiant. A titre d'exemple, on peut citer le carbonate de propylène, la γ-butyrolactone, les éther-esters des mono-, di-, tri- éthylène ou propylène glycols, les éther-alcools des mono-, di-, tri- éthylène ou propylène glycols, les plastifiants tels que les esters de l'acide phtalique ou de l'acide citrique.

Dans un autre mode de mise en oeuvre de l'invention, on utilise comme solvant ou diluant un composé réactif vis-à-vis de la polymérisation, qui est un composé de faible masse moléculaire et de faible viscosité qui va jouer à la fois le rôle de monomère polymérisable et le rôle de solvant ou de diluant pour des monomères plus visqueux ou des prépolymères utilisés conjointement. Après la réaction, ces monomères ayant servi de solvant font partie du réseau macromoléculaire finalement obtenu, leur intégration étant plus grande lorsqu'il s'agit de monomères bi-fonctionnels. Le matériau obtenu après irradiation ne contient plus de produits ayant un faible poids moléculaire et une tension de vapeur appréciable, ou susceptibles de contaminer les objets avec lesquels le polymère est en contact. A titre d'exemple, un solvant réactif peut être choisi parmi les mono et di éthers vinyliques des mono-, di-, tri-, tétra- éthylène et propylène glycols, le trivinyl éther triméthylolpropane, le divinyléther du diméthanol-cyclohexane, la N-méthylpyrolidone, le 2-propényléther du carbonate de propylène commercialisé par exemple sous la dénomination PEPC par la société ISP, New Jersey, Etats-Unis.

Pour irradier le mélange réactionnel, le rayonnement peut être choisi parmi le rayonnement ultraviolet, le rayonnement visible, les rayons X, les rayons γ et le rayonnement β. Lorsque l'on utilise la lumière ultraviolette comme rayonnement actinique, il peut être avantageux d'ajouter aux photoinitiateurs de l'invention des photosensibilisateurs destinés à permettre une photolyse efficace avec les longueurs d'ondes moins énergétiques que celles correspondant au maximum d'absorption du photoinitiateur, telles que celles émises par les dispositifs industriels, (λ ≈ 300 nm pour les lampes à vapeur de mercure en particulier). De tels additifs sont connus, et à titre d'exemples non limitatifs, on peut citer l'anthracène, le diphényl-9,10-anthracène, le pérylène, la phénothiazine, le tétracène, la xanthone, la thioxanthone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles permettant entre autre de changer la longueur d'onde d'absorption. L'isopropylthioxantone est un exemple de photosensibilisateur préféré lorsque l'on utilise un sel d'iodonium selon l'invention comme photoinitiateur.

Parmi les différents types de rayonnement mentionnés, le rayonnement ultraviolet est particulière préféré. D'une part, il est plus commode d'emploi que les autres rayonnements mentionnés. D'autre part, les photoinitiateurs sont en général directement sensibles aux rayons UV et les photosensibilisateurs sont d'autant plus efficaces que la différence d'énergie (δλ) est plus faible.

Les composés ioniques de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie- radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants. Les éthers vinyliques ne sont pas ou sont peu actifs par amorçage radicalaire. Il est donc possible, dans un mélange réactionnel contenant un photoinitiateur selon l'invention, un amorceur radicalaire, au moins un monomère du type éther vinylique et au moins un monomère comprenant des doubles liaisons non activées telles que celles des groupes allyliques, d'effectuer une polymérisation séparée de chaque type de monomère. Il est par contre connu que les monomères déficients en électrons, tels que les esters ou les amides de l'acide fumarique, de l'acide maléique, de l'acide acrylique ou méthacrylique, de l'acide itaconique, de l'acrylonitrile, du méthacrylonitrile, la maléimide et ses dérivés, forment en présence d'éthers vinyliques riches en électrons, des complexes de transfert de charge donnant des polymères alternés 1:1 par amorçage radicalaire. Un excès initial de monomères vinyliques par rapport à cette stoechiométrie permet de préserver des fonctions polymérisables par initiation cationique pure. Le déclenchement de l'activité d'un mélange d'amorceur radicalaire et d'amorceur, cationique selon l'invention peut être fait simultanément pour les deux réactifs dans le cas par exemple d'insolation par un rayonnement actinique d'une longueur d'onde pour laquelle les photoinitiateurs de l'invention et les amorceurs radicalaires choisis sont actifs, par exemple à λ = 250 nm. A titre d'exemple, on peut citer comme amorceurs les produits commerciaux suivants : Irgacure 184^{®}, Irgacure 651^{®}, Irgacure 261^{®} Quantacure DMB^{®}, Quantacure ITX^{®}.

Il peut aussi être avantageux d'utiliser les deux modes de polymérisation d'une manière séquentielle, pour former dans un premier temps des prépolymères dont la mise en forme est aisée et dont le durcissement, l'adhésion, la solubilité ainsi que le degré de réticulation peuvent être modifiés par le déclenchement de l'activité de l'amorceur cationique. Par exemple, un mélange d'un amorceur radicalaire thermodissociable et d'un photoinitiateur cationique selon l'invention permet de réaliser des polymérisations ou des réticulations séquentielles, d'abord sous l'action de la chaleur, puis sous l'action d'un rayonnement actinique. D'une manière similaire, si l'on choisit un amorceur radicalaire et un photoinitiateur cationique selon l'invention, le premier étant photosensible à des longueurs d'ondes plus longues que celle déclenchant le photoinitiateur selon l'invention, on obtient une réticulation en deux étapes contrôlables. Des amorceurs radicalaires peuvent être par exemple Irgacure® 651 permettant d'amorcer des polymérisations radicalaires à des longueurs d'onde de 365 nm.

L'invention a également pour objet l'utilisation des composés ioniques de l'invention pour les réactions d'amplification chimique de photoresists pour la microlithographie. Lors d'une telle utilisation, un film d'un matériau comprenant un polymère et un composé ionique de l'invention est soumis à une irradiation. L'irradiation provoque la formation de l'acide par remplacement du cation M par un proton, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser un composé de l'invention qui se présente sous la forme d'un polymère constitué essentiellement d'unités récurrentes styrényles portant un substituant ionique -C(CN)₂⁻. Ces composés permettent d'obtenir après photolyse des produits qui ne sont pas volatils, et donc pas odorants lorsqu'il s'agit de sulfures. Parmi les polymères qui peuvent ainsi être modifiés en présence d'un composé de l'invention, on peut citer notamment les polymères contenant des motifs ester ou des motifs aryléther de tertioalkyle, par exemple les poly(phtalaldéhydes), les polymères de bisphénol A et d'un diacide, le polytertiobutoxycarbonyl oxystyrène, le polytertiobutoxy-α-méthyl styrène, le polyditertiobutylfumarate-co-allyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Les composés ioniques de la présente invention, qui présentent une grande stabilité thermique, offrent de nombreux avantages par rapport aux sels connus de l'art antérieur. Ils ont des vitesses d'amorçage et de propagation comparables ou supérieures à celles obtenues à l'aide des anions de coordination de type PF₆⁻, AsF₆⁻ et surtout SbF₆⁻. En outre, le coefficient de diffusion de l'anion -C(CN)₂⁻. est supérieur à celui des anions hexafluorométallates ou des anions tétrafluoroborates ou des anions phénylborates. Ces propriétés sont expliquées par la délocalisation de la charge négative et la faible répulsion entre les charges partielles portées par les azotes des groupements nitriles et éloignées l'une de l'autre de 2Å.

Dans les composés de la présente invention, les paires d'ions présentent un très forte dissociation, ce qui permet l'expression des propriétés catalytiques intrinsèques du cation M^{m+}, dont les orbitales actives sont facilement exposées aux substrats de la réaction, ceci dans des milieux variés. La plupart des réactions importantes de la chimie organique (peuvent être ainsi effectuées dans des conditions peu contraignantes, avec d'excellents rendements et la facilité de séparer le catalyseur du milieu réactionnel. La mise en évidence d'induction asymétrique par l'utilisation d'un composé ionique selon l'invention qui portent un groupement chiral est particulièrement importante de part sa généralité et sa facilité de mise en oeuvre. La présente invention a par conséquent comme autre objet l'utilisation des composés de l'invention comme catalyseurs dans les réactions de Friedel-Crafts, les réactions de Diels-Alder, les réactions d'aldolisation, les additions de Michael, les réactions d'allylation, les réactions de couplage pinacolique, les réactions de glycosilation, les réactions d'ouvertures de cycle des oxétanes, les réactions de méthathèse des alcènes, les polymérisations de type Ziegler-Natta, les polymérisations du type méthathèse par ouverture de cycle et les polymérisations du type méthathèse de diènes acycliques. Les composés ioniques de l'invention préférés pour une utilisation en tant que catalyseur pour les réactions ci-dessus sont ceux dans lesquels le cation est choisi parmi le lithium, le magnésium, le cuivre, le zinc, l'étain, les métaux trivalents, y compris les terres rares, les platinoïdes, et leurs couples organométalliques, en particulier les métallocènes.

Les composés de l'invention peuvent également être utilisés comme solvant pour effectuer des réactions chimiques, photochimiques, électrochimiques, photoélectrochimiques. Pour cette utilisation particulière, on préfère les composés ioniques dans lesquels le cation est un imidazolium, un triazolium, un pyridinium ou un 4-diméthylamino-pyridinium, ledit cation portant éventuellement un substituant sur les atomes de carbone du cycle. Les composés étant utilisés sous leur forme liquide, on préfère tout spécialement ceux qui ont un point de fusion inférieur à 150°C, plus particulièrement inférieur à 100°C.

Les inventeurs ont également trouvé que la charge anionique portée par le groupement -C(CN)₂⁻ exerçait un effet stabilisant sur les conducteurs électroniques de type polymères conjugués, et que l'utilisation d'un composé dans lequel le substituant Z comprenait une longue chaîne alkyle permettait, de rendre ces polymères solubles dans les solvants organiques usuels même à l'état dopé. Le greffage de ces charges sur le polymère lui-même donne des polymères à charge globale cationique, solubles et présentant, outre leur stabilité, des propriétés d'anticorrosion vis-à-vis des métaux, tels que l'aluminium et les métaux ferreux. La présente invention a pour objet des matériaux à conduction électronique comprenant un composé ionique de la présente invention dans lequel la partie cationique est un polyçation constitué par un polymère conjugué dopé "p". Les composés ioniques préférés pour cette application sont ceux dans lesquels le substituant Z ou R_{D} contient une chaîne alkyle ayant de 6 à 20 atomes de carbone.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de composés ioniques de l'invention pour la fabrication de colorants cationiques dont les contre ions, éventuellement fixés à cette même molécule, correspondent aux fonctionnalités de l'invention, permet de réduire les phénomènes d'agrégation, y compris dans des matrices solides polymères et de stabiliser ces colorants. La présente invention a pour autre objet une composition de colorant cationique, caractérisée en ce qu'elle contient un composé ionique selon l'invention. Les composés ioniques particulièrement préférés pour cette application sont ceux dans lesquels la ou les charges négatives du groupement anionique -C(CN)₂⁻ sont soit fixées à la molécule de colorant, soit elles constituent le contre-ion des charges positives du colorant.

Quelques exemples de composés selon l'invention sont donnés ci-après :

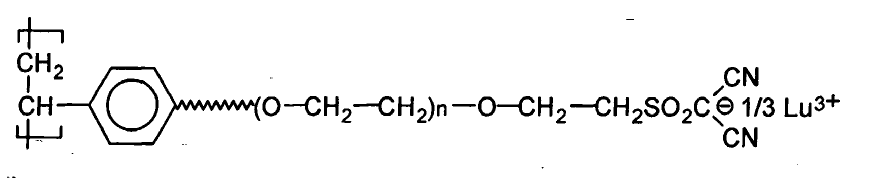

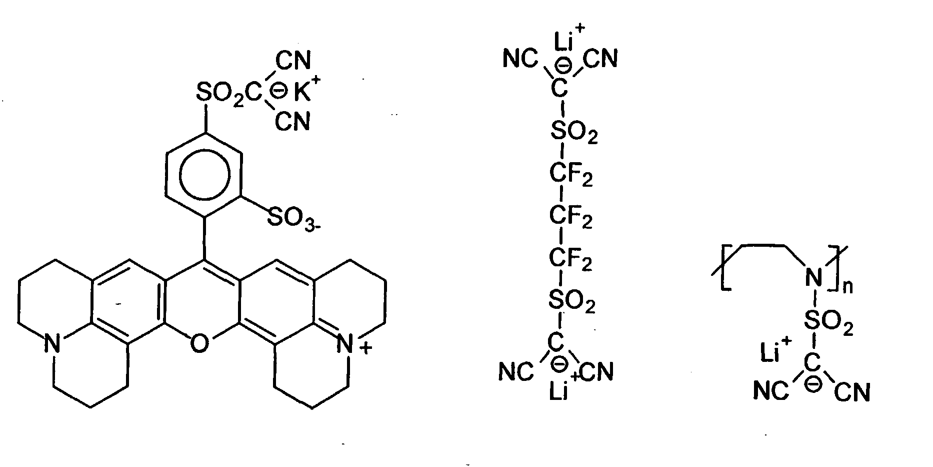

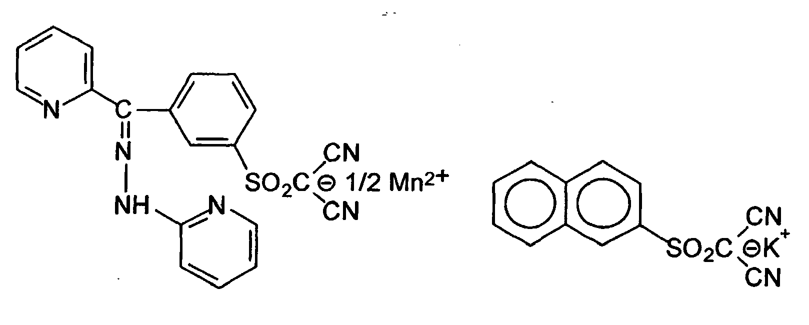

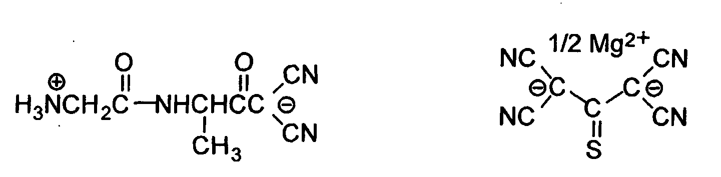

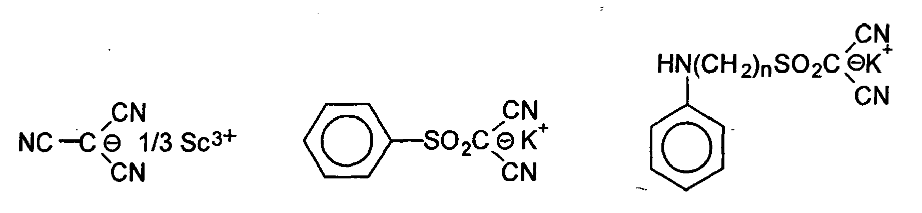

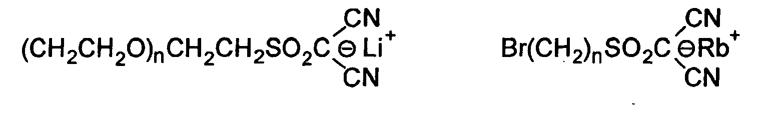

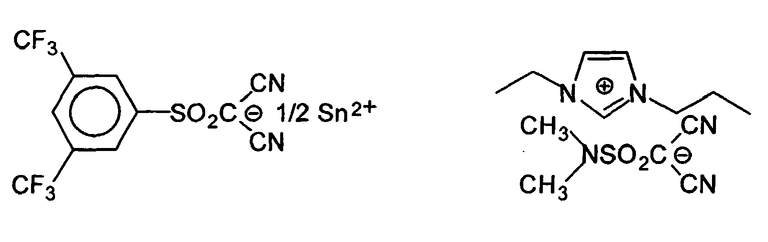

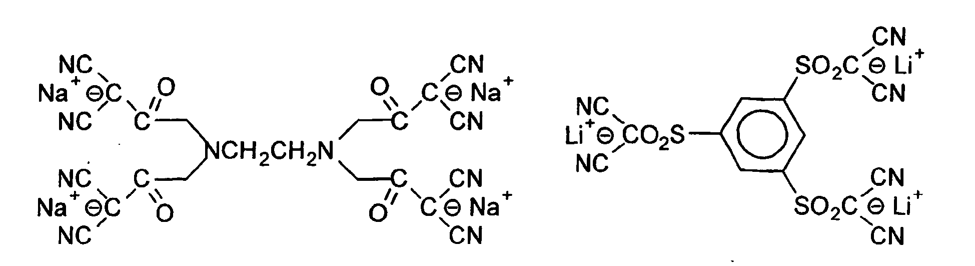

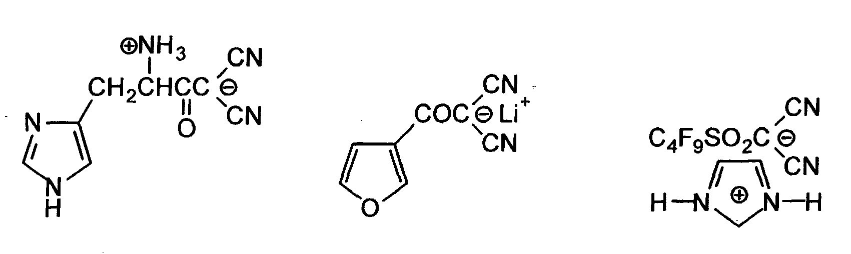

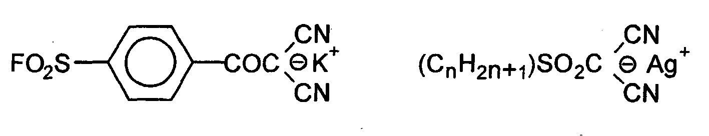

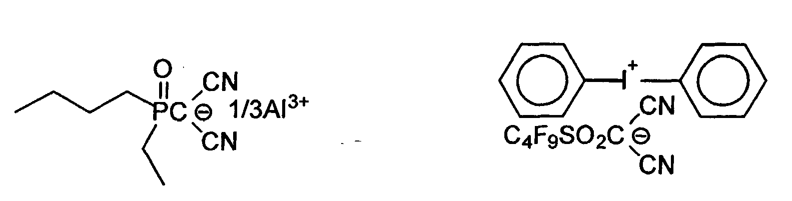

La présente invention est décrite plus en détails par les exemples suivants, qui décrivent la préparation et diverses utilisations de composés de l'invention.

Tous les composés selon l'invention ont été préparés à partir de sels de métal alcalin du malononitrile. Lesdits sels ont été obtenus à partir de malononitrile commercial purifié au préalable dans une cellule de sublimation à 40°C sous vide secondaire, le malononitrile étant récupéré après 48 heures sur le doigt froid de la cellule, sous forme de cristaux blancs qui sont ensuite conservés sous argon.

On a obtenu le sel de lithium en dosant une solution aqueuse de malononitrile par une solution titrée d'hydroxyde de lithium LiOH, le point de neutralisation étant déterminé à l'aide d'un pH-mètre. La solution aqueuse a ensuite été lyophilisée, puis le produit séché sous vide secondaire à l'ambiante pendant 72 heures. On a ainsi obtenu le sel de lithium qui, conservé sous argon, a une pureté déterminée par RMN du proton et du carbone supérieure à 99%.

On a obtenu par le même procédé les sels de sodium et de potassium en remplaçant l'hydroxyde de lithium respectivement par l'hydroxyde de sodium et l'hydroxyde de potassium.

### Exemple 1

324 mg (1 mmole) de chlorure de 4-(diméthylamino)-azobenzène-4'-sulfonyle dans 5 ml de tétrahydrofurane ont été ajoutés à 104 mg (1 mmole) du sel de potassium du malononitrile dans 5 ml de THF et 500 µl de triéthylamine. Après 24 heures sous agitation, le précipité de chlorure de potassium a été éliminé et, après évaporation du solvant, on a obtenu le sel de triéthylammonium qui a été mis en suspension dans 5 ml d'une solution aqueuse contenant 350 mg de bromure de tétrabutylammonium. Le mélange a été agité pendant 24 heures. On a obtenu une poudre de couleur orange qui a une pureté, caractérisée par RMN du proton et du carbone, supérieure à 98%, qui est soluble dans la plupart des solvants organiques, et qui répond à la formule suivante :

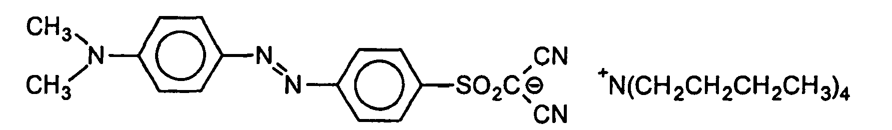

Ce colorant ionique est un indicateur de pH en milieu non aqueux (transition jaune-orange - rouge-violet dans la zone de pH 1-4).

### Exemple 2 (de référence seulement)

En opérant dans une boîte à gants sous argon, à 24,15 g (100 mmoles) de di-2-éthylhexylamine dans 100 ml de THF à -20°C, on a ajouté par portions 32 ml de butyllithium 2M dans le cyclohexane (100 mmoles). Après une heure, on a ajouté 11,85 g (100 mmoles) de fluorure de chlorosulfonyle FSO₂Cl. La réaction s'est poursuivie 4 heures à -20°C, puis pendant 24 heures à température ambiante. On a alors porté la température à 0°C et on ajouté 10,42 g (100 mmoles) du sel de potassium du malononitrile KHC(CN)₂ et 11,22 g (100 mmoles) de DABCO. Après 24 heures à 0°C, le milieu réactionnel a été filtré pour éliminer le précipité de chlorure de potassium et l'hydrochlorure de DABCO. Après évaporation du solvant et séchage, on a récupéré le sel de lithium du di-2-éthylhexylaminosulfonylmalononitrile, ayant une pureté, caractérisée par RMN du proton et du carbone, supérieure à 98%.

Suivant le même procédé, on a obtenu le sel de lithium du dibutylaminosulfonylmalononitrile à partir de la dibutylamine.

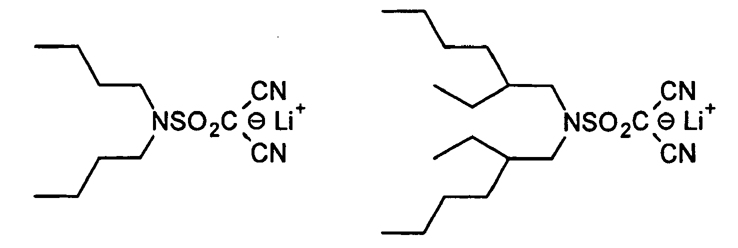

Les sels de potassium ont été obtenus en traitant les sels de lithium dans le minimum d'eau par le fluorure de potassium KF. Après filtration, évaporation et séchage, on a récupéré quantitativement les sels de potassium.

Ces sels sont solubles dans la plupart des solvants organiques usuels (tétrahydrofurane, acétonitrile, diméthylformamide, acétate d'éthyle, glymes) et dans les polymères solvatants aprotiques.

### Exemple 3

5,44 g (10 mmoles) de iodure de 3,3'-diéthylthiatricarbocyanine (commercialisé par Aldrich, Milwaukee, USA) et 4,08 g (10 mmoles) du sel de potassium du di-2-éthylhexylaminosulfonylmalononitrile préparé selon l'exemple 2 ont été agités ensemble durant 24 heures dans l'eau. Par extraction de la phase aqueuse par du dichlorométhane, on a récupéré la 3,3'-diéthylthiatricarbocyanine du di-2-éthylhexylaminosulfonylmalononitrile, ayant une pureté, caractérisée par RMN du proton supérieure, à 99%.

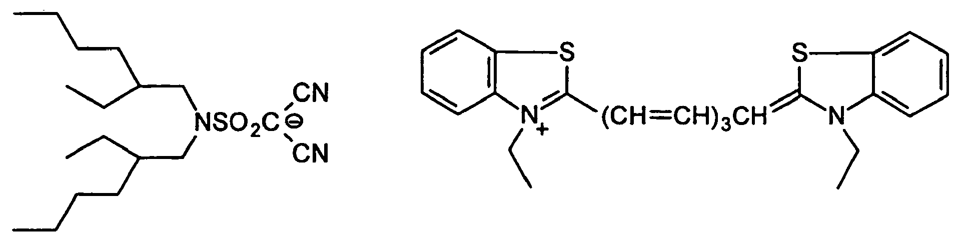

Ce sel est très soluble dans les solvants peu polaires comme le dichlorométhane ou le chlorure de méthylène ainsi que dans les matrices polymères peu polaires comme le polyméthacrylate de méthyle.

Lorsqu'il est utilisé comme colorant, on constate une très nette diminution de l'agrégation des molécules de colorant cationique entre elles, du fait du caractère "plastifiant" des différents groupements dialkylamino, ce qui constitue un avantage. En effet, le phénomène d'agrégation est préjudiciable au bon fonctionnement des systèmes utilisant des colorants, en particulier dans les disques optique de stockage d'informations, car il provoque un élargissement des bandes d'absorption optiques.

### Exemple 4

3,17 g (10 mmoles) de chlorure de diphényliodonium (C₆H₅)₂ICl et 4,08 g (10 mmoles) de sel de potassium di-2-éthylhexylaminosulfonylmalononitrile préparé selon l'exemple 2 ont été agités ensemble durant 24 heures dans l'eau. Par extraction de la phase aqueuse par du dichlorométhane, on a récupéré le diphényliodonium du di-2-éthylhexylaminosulfonylmalononitrile, ayant une pureté, caractérisée par RMN du proton, supérieure à 99%.

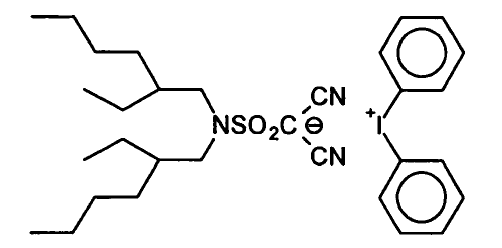

Ce sel permet d'amorcer, sous l'effet d'un rayonnement actinique (lumière, rayons γ, faisceaux d'électrons), la réaction de réticulation cationique de monomères riches en électrons (éthers vinyliques, propylvinyléthers).

Il est soluble dans la plupart des solvants organiques usuels (tétrahydrofurane, acétonitrile, diméthylformamide, acétate d'éthyle, glymes) et dans les polymères solvatants aprotiques comme le polyoxyde d'éthylène. Il est aussi soluble à plus de 5% en poids dans les solvants réactifs comme le triéthylèneglycol divinyl éther, contrairement par exemple au sel de bis(trifluorométhanesulfonyl)imide du diphényliodonium.

On a testé les propriétés de photo-amorçage de ce sel en irradiant par un rayonnement U.V. à 254 nm, d'une puissance de 1900 mW/cm², un feutre non tissé de polyéthylène imbibé de triéthylèneglycol divinyl éther (79% en poids) contenant le diphényliodonium du di-2-éthylhexylaminosulfonylmalononitrile (1% en poids) du présent exemple et le 7,8-octène-3,6-oxa-1-sulfonylmalononitrile (20% en poids). Après une période de quelques secondes sous irradiation, suivie d'une période de 10 min permettant la propagation des espèces générées dans le milieu (postcure), on a obtenu le polyélectrolyte supporté par le feutre. Ce type de composite est très intéressant pour le développement de batteries au lithium à électrolyte polymère ou gel.

### Exemple 5

5,91 g (20 mmoles) du sel de potassium du dibutylaminosulfonylmalononitrile, préparés selon l'exemple **2**, ont été mis en solution dans 10 ml d'eau. On a ajouté, sous agitation, 2,71 g (10 mmoles) d'hydrochlorure de 2,2'-azobis(2-méthylpropionamidine) [=NC(CH₃)₂C(=NH)NH₂]₂•2 HCl (commercialisé par Aldrich) en solution dans 10 ml d'eau. Il s'est formé immédiatement un précipité qui a été recueilli par filtration. Après séchage sous vide à température ambiante, on a récupéré le dibutylaminosulfonylmalononitrile de 2,2'-azobis(2-méthylpropionamidine).

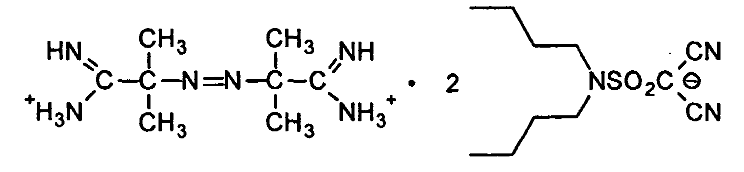

Ce sel est un amorceur de polymérisation radicalaire soluble dans la plupart des solvants organiques usuels (tétrahydrofurane, acétonitrile, diméthylformamide, acétate d'éthyle, glymes) et dans les monomères et polymères solvatants aprotiques, contrairement à l'hydrochlorure de 2,2'-azobis(2-méthylpropionamidine).

On a préparé une solution dans l'acétonitrile de 1 partie de cet initiateur et de 100 parties d'un polymère contenant des insaturations éthyléniques, obtenu par polycondensation de polyéthylène glycol de masse 1000 avec le 3-chloro-2-chlorométhyl-1-propène selon la procédure décrite par Alloin, et al (*Solid States Ionics,* (1993), 60, 3). On a coulé la solution visqueuse obtenue sur un film de polypropylène (PP). Après évaporation du solvant, le film de polymère d'une épaisseur de 110 µm sur PP a été stocké une semaine en boîte à gants sous argon pour le sécher. La réticulation a alors été initiée en portant la température du film à 60°C. Après une nuit, on a obtenu un film présentant de bonnes propriétés mécaniques et un faible taux d'extractibles (inférieure à 1%). La solubilité de l'initiateur utilisé dans la matrice polymère permet donc d'obtenir une réticulation efficace et homogène. De plus, cet initiateur, contrairement par exemple au 2,2'-azobisisobutyronitrile, n'est pas volatil et la quantité ajoutée peut être optimisée au mieux pour chaque type de polymérisation.

### Exemple 6

A une solution dans 10 ml de tétrahydrofurane anhydre à 0°C de 4,49 g (40 mmoles) de DABCO et de 1,32 g (20 mmoles) de malononitrile, on a ajouté 6,05 g (20 mmoles) de chlorure de 4-iodobenzènesulfonyle IC₆H₄SO₂Cl (commercialisé par Aldrich) dilués dans 5 ml de tétrahydrofurane anhydre. Après deux heures à 0°C, la réaction s'est poursuivie durant 24 heures à l'ambiante. L'hydrochlorure de DABCO formé au cours de la réaction a été éliminé par filtration sur un verre fritté de porosité N°4. Après évaporation de l'acétonitrile de la solution filtrée, le produit a été repris dans 15 ml d'eau froide et l'on a ajouté lentement 1,49 g (20 mmoles) de chlorure de potassium anhydre en solution dans 5 ml d'eau. Il est apparu un précipité qui a été recueilli par filtration sur un verre fritté de porosité N°4. Après séchage, on a récupéré le sel de potassium du 4-iodobénzènesulfonylmalononitrile.

Ce composé a été oxydé en iodosoacétate K[(NC)₂CSO₂C₆H₄I(O₂CCH₃)₂] par le mélange acide acétique, anhydride acétique et peroxyde d'hydrogène selon la méthode de Yamada & al (*Die Makromolecular Chemie,* (1972), 152, 153-162). 4,88 g (10 mmoles) dudit iodosoacétate ont été mis en suspension dans un mélange de 15 ml d'acide méthanesulfonique et de 4,51 g (30 mmoles) de butoxybenzène maintenu à 0°C pendant 4 heures. Le produit de réaction a été versé dans 300 ml d'éther et le précipité séparé par filtration, lavé par de l'éther et séché. On a ainsi obtenu 3,9 g du zwitterion (4-butoxybenzène)-(4-phénylsulphonylmalononitrile] iodonium (75% de rendement) ayant une pureté, caractérisée par RMN du proton et du carbone, supérieure à 97%.

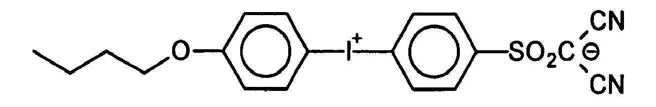

Ce zwitterion permet d'amorcer sous l'effet d'un rayonnement actinique (lumière, rayons γ, faisceaux d'électrons) la réaction de réticulation cationique de monomères riches en électrons (éthers vinyliques, propènyléthers vinyliques).

Il a une bonne solubilité dans la plupart des solvants organiques usuels (tétrahydrofurane, acétonitrile, diméthylformamide, acétate d'éthyle, glymes) et dans les monomères et les polymères solvatants aprotiques comme le polyoxyde d'éthylène. Il est aussi soluble à plus de 5% en poids dans les solvants réactifs comme le triéthylèneglycol divinyl éther.

On a testé les propriétés de photo-amorçage de ce sel en irradiant par un rayonnement U.V. à 254 nm, d'une puissance de 1900 mW/cm², une solution de triéthylèneglycol divinyl éther contenant à 1% en poids dudit sel d'iodonium. Après quelques secondes sous irradiation, le solvant réactif a pris en masse, cette réaction étant très exothermique.

### Exemple 7 (de référence seulement)

13,44 g (50 mmoles) de chlorure de 1-dodécanesulfonyle C₁₂H₂₅SO₂Cl (commercialisé par Lancaster) en solution dans 30 ml de tétrahydrofurane anhydre à -20°C ont été traités par 8,8 g (100 mmoles) du sel de sodium du malononitrile. Après 1 heure à 0°C, puis 24 heures à température ambiante, le solvant a été évaporé et le produit repris dans 30 ml d'eau. L'addition de 3,73 g (50 mmoles) de chlorure de potassium KCl anhydre a permis d'obtenir un précipité qui a été recristallisé, puis récupéré par filtration et enfin séché. On a obtenu le sel de potassium du 1-dodécanesulfonylmalononitrile, sous forme d'une poudre cristallisée, ayant une pureté, déterminée par RMN du proton et du carbone supérieure, à 99%.

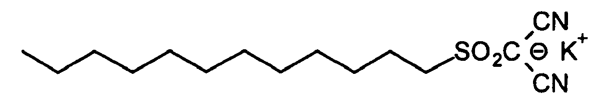

On a obtenu par un procédé similaire, les sels de potassium du 1-butanesulfonylmalononitrile et du 1-octylsulfonylmalononitrile à partir respectivement du chlorure de 1-butanesulfonyle et du chlorure de 1-octanesulfonyle.

Les sels de lithium de ces trois dérivés ont été préparés quantitativement par échange ionique entre le sel de potassium et le chlorure de lithium dans le tétrahydrofurane anhydre.

Le sel de lithium du 1-dodécanesulfonylmalononitrile dissous dans une matrice de polyoxyde d'éthylène à une concentration O/Li = 16/1 a un nombre de transport cationique ≈0,55. Il en résulte que, lorsque ce composé est utilisé dans l'électrolyte d'une batterie au lithium à électrolyte polymère, les gradients de concentration qui apparaissent au cours du fonctionnement de la batterie sont diminués de façon substantielle. Les performances lors d'appels de puissance sont ainsi améliorées.

Les sels du 1-dodécanesulfonylmalononitrile présentaient un intérêt indéniable comme additif pour le laminage du lithium et pour l'extrusion de polymères, en particulier l'extrusion du polyoxyde d'éthylène.

### Exemple 8 (de référence seulement)

Dans 100 ml d'eau à 0°C, on a ajouté 23,01 g (100 mmoles) d'hexafluoropropanesultone (commercialisé par Fluorochem). Après 2 heures sous agitation, la phase aqueuse a été extraite par deux fractions de 20 ml de dichlorométhane, puis les phases organiques ont été réunies et séchées par du sulfate de magnésium. Après évaporation du dichlorométhane et distillation du liquide recueilli, on a obtenu 16,94 g de fluorure de 1-fluoro-2,2,2-trifluoroéthanesulfonyle CF₃CHFSO₂F (rendement : 94% ; pureté, déterminée par RMN du proton et du fluor, supérieure à 99%).

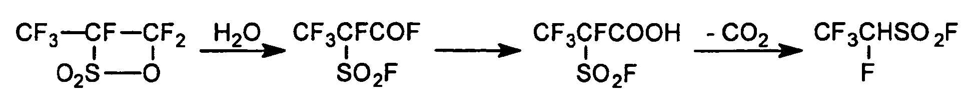

En opérant dans une boîte à gants sous argon, on a mis en solution 9,2 g (50 mmoles) du composé ainsi préparé dans 10 ml de tétrahydrofurane anhydre. Après avoir porté cette solution à -20°C, on a ajouté lentement 50 ml d'une solution 1 M (50 mmoles) dans le tétrahydrofurane de tert-butoxide de potassium (CH₃)₃COK (commercialisé par Aldrich). Après 15 min, on a ajouté 12,46 g (50 mmoles) de 1-bromododécane. La réaction s'est poursuivie pendant 2 heures à -20°C, puis pendant 24 heures à température ambiante. On a alors ajouté 8,8 g (100 mmoles) du sel de sodium du malononitrile. Après 48 heures, le solvant a été évaporé et le résidu recristallisé dans 50 ml d'eau contenant 7,46 g (100 mmoles) de chlorure de potassium KCl. Après filtration et séchage, on a obtenu le sel de potassium du 1-dodécane-2,2,2-trifluoroéthanesulfonylmalononitrile ayant une pureté, caractérisée par RMN du proton, du carbone et du fluor, supérieure à 99%.

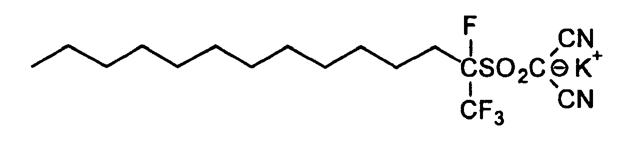

On a obtenu quantitativement le sel de lithium en traitant le sel de potassium dans le tétrahydrofurane anhydre par la quantité stoechiométrique de chlorure de lithium anhydre, filtration du milieu réactionnel, évaporation du solvant et séchage sous vide.

Ces composés peuvent être utilisés comme additif pour le laminage du lithium et pour l'extrusion de polymères, en particulier l'extrusion du polyoxyde d'éthylène. Ils présentent aussi des propriétés plastifiantes et antistatiques.

### Exemple 9

A 6,61 g de malononitrile (100 mmoles) en solution dans 50 ml de THF à -20°C, on a ajouté par portions 795 mg d'hydrure de lithium LiH. Après deux heures à -20°C, on a ajouté 20,14 g (100 mmoles) de 1-(trifluorométhanesulfonyl)-imidazole (commercialisé par Fluka). La réaction s'est poursuivie pendant 4 heures à -20°C, puis 48 heures à température ambiante. Le solvant a alors été évaporé et le résidu repris dans 60 ml d'eau. On a ensuite ajouté 14,66 g (100 mmoles) du chlorure de 1-éthyl-3-méthyl-1*H*-imidazolium (commercialisé par Aldrich) à la solution aqueuse. Il s'est immédiatement formé une phase plus dense que l'eau. Cette phase a été récupérée par extraction au dichlorométhane. Après évaporation du dichlorométhane et séchage sous vide à 40°C du liquide obtenu, on a récupéré le sel fondu de 1-éthyl-3-méthyl-1*H*-imidazolium du trifluorométhanesulfonylmalononitrile, avec une pureté, caractérisée par RMN du proton, du carbone et du fluor, supérieure à 98%.

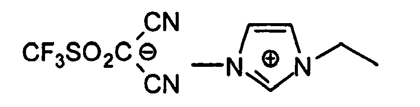

Ce sel fondu présente une conductivité de 4,5.10⁻³ S.cm⁻¹ et un point de congélation inférieure à -20°C. Son large domaine de stabilité rédox en fait un électrolyte particulièrement intéressant pour les générateurs électrochimiques tels que les batteries au lithium, les supercapacités, les systèmes de modulation de la lumière, les cellules photovoltaïques.

On a réalisé une supercapacité électrochimique en utilisant le sel fondu de 1-éthyl-3-méthyl-1*H*-imidazolium du trifluorométhanesulfonylmalononitrile comme électrolyte et des composites carbone/aluminium comme électrodes. Les électrodes d'une épaisseur de 150 µm ont été placées de part et d'autre d'un polyéthylène microporeux ayant une épaisseur de 40 µm et le système complet a été scellé en boîte à gants dans un boitier de pile bouton après avoir été imbibé du sel fondu liquide. La supercapacité obtenue a permis de réaliser plus de 100.000 cycles de charge/décharge entre 0 et 2,5 V pour une densité d'énergie supérieure à 25 Wh/l et une puissance délivrée supérieure à 1500 W/l.

### Exemple 10

A 18,11 g (100 mmoles) de 6-bromo-1-hexanol et 11,22 g (100 mmoles) de DABCO dans 100 ml de THF anhydre à -20°C, on a ajouté lentement 19,06 g (100 mmoles) de chlorure de tosyle. Après 24 heures sous agitation à -20°C, le milieu réactionnel a été filtré pour éliminer le précipité d'hydrochlorure de DABCO. Après évaporation du solvant, on a récupéré quantitativement le tosylate du 6-bromo-1-hexanol CH₃FSO₂O(CH₂)₆Br. Ce composé a ensuite été dissous dans 200 ml de THF avec 40 g d'aniline FNH₂ (200 mmoles) et cette solution a été portée au reflux pendant une nuit. Après refroidissement, on a ajouté 300 ml d'eau et la phase organique a été extraite avec de l'éther. Après avoir été lavée avec de l'eau, la phase éthérée a été séchée par du sulfate de magnésium. On a obtenu après évaporation et séchage, 23 g de la N-(6-bromohexyl)aniline.

En opérant dans une boîte à gants sous argon, on a mis en solution 9,2 g de fluorure de 1-fluoro-2,2,2-trifluoroéthanesulfonyle CF₃CHFSO₂F (50 mmoles), préparé conformément à l'exemple 8, dans 10 ml de tétrahydrofurane anhydre. Après avoir porté cette solution à -20°C, on a ajouté lentement 50 ml d'une solution 1 M dans le tétrahydrofurane de tert-butoxide de potassium (CH₃)₃COK (commercialisé par Aldrich). Après 15 min, on a ajouté 12,81 g (50 mmoles) de N-(6-bromohexyl)aniline. La réaction s'est poursuivie pendant deux heures à -20°C, puis pendant 24 heures à température ambiante. On a alors ajouté 8,8 g (50 mmoles) du sel de sodium du malononitrile et 5,61 g de DABCO. Après 48 heures, le milieu réactionnel a été filtré pour éliminer le précipité d'hydrochlorure de DABCO, puis le solvant a été évaporé et le résidu recristallisé dans 50 ml d'eau contenant 7,46 g (100 mmoles) de chlorure de potassium KCl. Après filtration et séchage, on a obtenu le sel de potassium du 1-(6-anilino-1-hexane)-2,2,2-trifluoroéthanesulfonylmalononitrile avec une pureté, caractérisée par RMN du proton, du carbone et du fluor, supérieure à 99%.

8,87 g de ce composé (20 mmoles) ont ensuite été dissous dans 200 ml d'une solution 1 M d'acide chlorhydrique, et on a ajouté lentement sur une période de 3 heures, 4,56 g (20 mmoles) de persulfate d'ammonium (NH₄)₂S₂O₈. La réaction s'est poursuivie pendant 14 heures. Le milieu réactionnel a alors été neutralisé par de l'hydroxyde de potassium, puis concentré à un volume de ≈40 ml. On a récupéré après filtration et séchage 5,9 g d'une poudre noire du composé suivant :

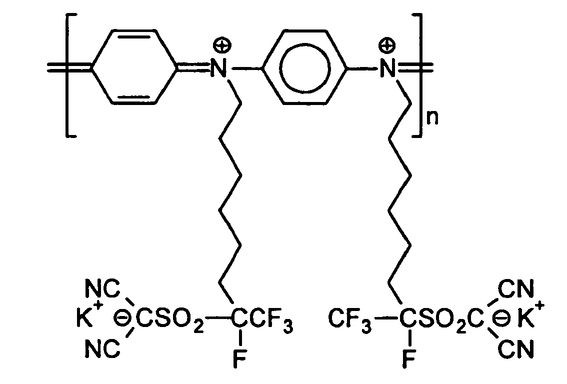

Ce composé polymère qui comprend un anion dopant très délocalisé dans sa structure, présente des propriétés de conducteur électronique (PCE). La faible basicité de cet anion améliore la stabilité du polymère. En milieu humide, cette conductivité déterminée par une mesure quatres points est initialement de l'ordre de 4 S.cm⁻¹.

On a testé ce matériau comme cathode de batterie. La batterie avait la constitution suivante :
- une cathode composite constituée par 40% en volume du composé polymère obtenu dans le présent exemple et 60% en volume de polyoxyde d'éthylène de masse 3.10⁵ ;
- un électrolyte constitué par un film de polyoxyde d'éthylène de masse 5.10⁶ contenant le sel de lithium de du butanesulfonylmalononitrile, obtenu à l'exemple 7, à une concentration à une concentration O/Li = 20/1 ;
- une anode de lithium métallique.

Après assemblage de l'ensemble dans un boîtier de pile bouton, la batterie obtenue a été cyclée à une température de 60°C entre 3 V et 3,9 V. Plus de 1000 cycles de charge/décharge ont pu être effectués en conservant 80% de la capacité du premier cycle.

En outre, le composé polymère du présent exemple est un bon inhibiteur de corrosion des métaux ferreux et permet de réaliser des dépôts sur des plastiques traités par effet Corona.

## Revendications

1. Composé ionique dérivé du malononitrile comprenant une partie anionique associée à au moins une partie cationique M^{m+} en nombre suffisant pour assurer la neutralité électronique de l'ensemble, **caractérisé en ce que** la partie anionique répond à l'une des formules R_{D}-Y-C(C≡N)₂⁻ ou Z-C(C≡N)₂⁻ dans lesquelles :
- Z représente un radical électro-attracteur ayant un paramètre de Hammett au moins égal à celui d'un radical phényle, choisi parmi :
j) -SCN, -N₃. -CF₃, FSO₂-, R'_{F}CH₂- (R'_{F} étant un radical perfluoré), les radicaux fluoroalkyloxy, fluoroalkylthioxy, fluoroalkényloxy, fluoroalkénylthioxy ;
jj) les radicaux comprenant un ou plusieurs noyaux aromatiques contenant éventuellement au moins un atome d'azote, d'oxygène, de soufre ou de phosphore, les dits noyaux pouvant être éventuellement des noyaux condensés et/ou lesdits noyaux pouvant éventuellement porter au moins un substituant choisi parmi les halogènes, -CN, -NO₂, -SCN, -N₃, CF₂=CF-O-, les radicaux R_{F}- et R_{F}-CH₂- dans lesquels R_{F} représente un radical perfluoroalkyle ayant de 1 à 12 atomes de carbone, les groupes fluoroalkyloxy, les groupes fluoroalkylthioxy, les radicaux alkyles, alkényles, oxaalkyles, oxaalkényles, azaalkyles, azaalkényles, thiaalkyles, thiaalkényles, les radicaux polymères, les radicaux possédant au moins un groupement ionophore cationique et/ou au moins un groupement ionophore anionique ;
- Y représente un groupement sulfonyle ou un groupement phosphonyle ;
- R_{D} est un radical choisi parmi : a) les radicaux alkyle ou alkényle, les radicaux aryle, arylalkyle, alkylaryle ou alkénylaryle, les radicaux alicycliques ou hétérocycliques, y compris les radicaux polycycliques ;
b) les radicaux alkyle ou alkényle comprenant au moins un groupe fonctionnel éther, thioéther, amine, imine, amide, carboxyle, carbonyle, isocyanate, isothiocyanate, thiocarbonyle, hydroxy ;
c) les radicaux aryle, arylalkyle, arylalkényle, alkylaryle ou alkénylaryle, dans lesquels les noyaux aromatiques et/ou au moins un substituant du noyau comprennent des hétéroatomes tels que l'azote, l'oxygène, le soufre ;
d) les radicaux comprenant des cycles aromatiques condensés qui comprennent éventuellement au moins un hétéroatome choisi parmi l'azote, l'oxygène, le soufre ;
e) les radicaux halogénés ou perhalogénés alkyle, alkényle, aryle, arylalkyle, alkylaryle, lesdits radicaux comprenant éventuellement des groupes fonctionnels éther, thioéther, imine, amine, carboxyle, carbonyle ou hydroxy ;
f) les radicaux R_{C}C(R')(R")-O- dans lesquels R_{C} est un radical alkylperfluoré et R' et R" sont indépendamment l'un de l'autre un atome d'hydrogène ou un radical tel que défini en a), b), c) ou d) ci-dessus;
g) les radicaux (R_{B})₂N-, dans lesquels les radicaux R_{B} identiques ou différents sont tels que définis en a), b), c), d) et e) ci-dessus, l'un des R_{B} pouvant être un atome d'hydrogène, ou bien les deux radicaux R_{B} forment ensemble un radical divalent qui forme un cycle avec N ;
h) les radicaux polymères,
i) les radicaux possédant un ou plusieurs groupements ionophores cationiques et/ou un ou plusieurs groupements ionophores anioniques ;
et **en ce que** le cation M^{m+} est un cation onium choisi dans le groupe constitué par les cation R₃O⁺ (oxonium), NR₄⁺ (ammonium), RC(NR₂)₂⁺ (amidinium), C(NR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium), les radicaux R représentant de manière indépendante un H ou un radical choisi dans le groupe constitué par :
- les radicaux alkyles, alkényles, oxa-alkyles, oxa-alkényles, aza-alkyles, aza-alkényles, thia-alkyles, thia-alkényles, aryles, arylalkyles, alkylaryles, alkényl-aryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore ;
étant entendu que :
• les composés C₆H₅SO₂C(CN)₂NH₄, C₆H₅SO₂C (CN)₂C₆H₅CH₂-SC(NH₂)NH, C₆H₅SO₂C(CN)₂C₆H₅NH₃, m-NO₂C₆H₅SO₂C(CN)₂(Et)₃NH, C₆H₅SO₂C(CN)₂C₅H₁₀NH et C₆H₅SO₂C(CN)₂C₅H₅NH sont exclus ;
• un substituant Z peut être un radical monovalent, une partie d'un radical ayant une valence égale à 2 portant deux groupements -C⁻(C≡N)₂' ou un segment d'un polymère ;.
• un substituant R_{D} peut être un radical monovalent, une partie d'un radical ayant une valence supérieure à 2 portant plusieurs groupements -Y-C⁻(C≡N)₂' ou un segment d'un polymère ;
• "segment d'un polymère" signifie unité récurrente éthylène portant éventuellement un substituant alkylène ou un substituant oxyalkénylène, unité récurrente oxyalkylène portant éventuellement un groupe méthylène (dérivée d'un 2,3-époxypropane), unité récurrente styrène portant éventuellement un substituant polyoxyéthylène, , unité récurrente azaéthylène, unité récurrente 2-(triéthoxysilyl)éthyl-co-O-[2-(triméthoxysilyl)éthyl]-O'-méthylpolyéthylène glycol, unité récurrente bis[3-triméthoxysilyl)-propyl]amino, unité récurrente méthyléthylsiloxane, unité récurrente acrylonitrile-co-4-styrényle, ou unité récurrente aniline portant un substituant fluoroalkyle ;
• lorsqu'un cation porte au moins deux radicaux R différents de H, lesdits radicaux peuvent former ensemble des cycles aromatiques ou non englobant éventuellement le centre portant la charge cationique ;
• "groupement ionophore cationique" signifie un groupement iodonium, sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation M⁺ ;
• "groupement ionophore anionique" signifie une fonction carboxylate (-CO₂⁻), une fonction sulfonate (-SO₃⁻), une fonction sulfonimide (-SO₂NSO₂-) ou une fonction sulfonamide (-SO₂N-).

2. Composé selon la revendication 1, **caractérisé en ce que** le cation onium fait partie du radical Z ou du radical R_{D}.

3. Composé selon la revendication 1, **caractérisé en ce que** le cation onium fait partie d'une unité récurrente d'un polymère.

4. Composé selon la revendication 1, **caractérisé en ce que** le cation M⁺ est un hétérocycle cationique à caractère aromatique, comportant au moins un atome d'azote alkylé dans le cycle.

5. Composé selon la revendication 4, **caractérisé en ce que** le cation est un imidazolium, un imidazolinium, un triazolium, un pyridinium, un 4-diméthylamino-pyridinium, lesdits cations portant éventuellement un substituant sur les atomes de carbone du cycle.

6. Composé selon la revendication 1, **caractérisé en ce que** le cation M est un groupement possédant une liaison -N=N, -N=N⁺, un groupement sulfonium, un groupe phosphonium, un groupement iodonium, ou un cation arène-ferrocénium substitué ou non, éventuellement incorporé dans une trame polymérique.

7. Composé selon la revendication 1, **caractérisé en ce que** le cation est un cation diaryliodonium, un cation dialkylaryliodonium, un cation triarylsulfonium, un cation trialkylaryle sulfonium, ou un cation phénacyl-dialkyl sulfonium substitué ou non.

8. Composé selon la revendication 1, **caractérisé en ce que** le cation aryliodonium, ou le cation alkylaryliodonium, ou le cation triarylsulfonium, ou le cation alkylaryle sulfonium, ou le cation phénacyl-dialkyl sulfonium font partie d'une chaîne polymère.

9. Composé selon la revendication 1, **caractérisé en ce que** M est un cation organique incorporant un groupement 2,2'[Azobis(2-2'-imidazolinio-2-yl)propane]²⁺ ou 2,2'-Azobis(2-amidiniopropane)²⁺.

10. Composé selon la revendication 1, **caractérisé en ce que** le substituant Z est choisi dans le groupe constitué par -OCₙF₂ₙ₋₁, -OC₂F₄H, -SCₙF₂ₙ₊₁ et -SC₂F₄H, -OCF=CF₂, -SCF=CF₂, n étant un nombre entier de 1 à 8.

11. Composé selon la revendication 1, **caractérisé en ce que** Z est un radical CₙF₂ₙ₊₁CH₂-, n étant un nombre entier de 1 à 8.

12. Composé selon la revendication 1, **caractérisé en ce que** R_{D} est choisi parmi les radicaux alkyles, alkényle, oxa-alkyle, oxa-alkényle, aza-alkyle, aza-alkényle, thia-alkyle ou thia-alkényle ayant de 1 à 24 atomes de carbone, ou parmi les radicaux aryle, arylalkyle, alkylaryle ou alkénylaryle ayant de 5 à 24 atomes de carbone.

13. Composé selon la revendication 1, **caractérisé en ce que** R_{D} est choisi parmi les radicaux alkyles ou alkényles ayant de 1 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine, un groupe carboxyle.

14. Composé selon la revendication 1, **caractérisé en ce que** R_{D} est choisi parmi les radicaux aryle, arylalkyle, alkylaryle ou alkénylaryle, dans lesquels les noyaux aromatiques et/ou leurs substituants comprennent des hétéroatomes tels que l'azote, l'oxygène, le soufre.

15. Composé selon la revendication 1, **caractérisé en ce que** R_{D} est partie d'un radical poly(oxyalkylène) ou d'un radical polystyrène.

16. Composé selon la revendication 1, **caractérisé en ce que** R_{D} est un radical possédant un groupement iodonium, un groupement sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation M⁺.

17. Composé selon la revendication 1, **caractérisé en ce que** R_{D} possède un ou plusieurs groupements ionophores anioniques constitués par une fonction carboxylate (-CO₂⁻), une fonction sulfonate (-SO₃⁻), une fonction sulfonimide (-SO₂NSO₂-) ou une fonction sulfonamide (-SO₂N-).

18. Composé selon l'une des revendications 1, **caractérisé en ce que** R_{D} comporte au moins une insaturation éthylénique et/ou un groupe condensable et/ou un groupe dissociable par voie thermique, par voie photochimique ou par dissociation ionique.

19. Composé selon l'une des revendications 1, **caractérisé en ce que** R_{D} représente un groupe mésomorphe ou un groupe chromophore ou un polymère conducteur électronique autodopé ou un alcoxysilane hydrolysable.

20. Composé selon la revendication 1, **caractérisé en ce que** R_{D} représente une unité récurrente d'une chaîne polymère.

21. Composé selon la revendication 1, **caractérisé en ce que** R_{D} comporte un groupement susceptible de piéger les radicaux libres.

22. Composé selon la revendication 1, **caractérisé en ce que** R_{D} comporte ou un dipôle dissociant.

23. Composé selon la revendication 1, **caractérisé en ce que** R_{D} comporte un couple rédox.

24. Composé selon la revendication 1, **caractérisé en ce que** R_{D} comporte ou un ligand complexant.

25. Composé selon la revendication 1, **caractérisé en ce que** R_{D}- est optiquement actif.

26. Composé selon la revendication 1, **caractérisé en ce que** R_{D} représente un radical ayant une valence v supérieure à deux, comportant à chacune de ses extrémités libres un groupe -C(C≡N)₂^{-.}

27. Procédé de polymérisation ou de réticulation de monomères ou de prépolymères capables de réagir par voie cationique, **caractérisé en ce que** l'on utilise un composé selon la revendication 1 comme photoinitiateur source d'acide catalysant la réaction.

28. Procédé selon la revendication 27, **caractérisé en ce que** le composé ionique est choisi parmi ceux dont le cation possède une liaison -N=N⁺, -N=N-, un groupement sulfonium, un groupement iodonium ou un cation arène-ferrocénium substitué ou non, éventuellement incorporé dans une trame polymérique.

29. Procédé selonla revendication 27, caratérisé en ce que le substituant R_{D} du composé ionique est un radical alkyle non substitué, un radical comprenant un groupe oxa ou un sulfone, un radical comprenant un groupement sulfoxyde, sulfone, oxyde de phosphine ou phosphonate.

30. Procédé selon la revendication 27, **caractérisé en ce que** les monomères sont choisis dans le groupe constitué par les composés qui comportent une fonction éther cyclique, une fonction thioéther cyclique ou une fonction amine cyclique, les composés vinyliques, les éthers vinyliques, les oxazolines, les lactones et les lactames.

31. Procédé selon la revendication 27, **caractérisé en ce que** le prépolymère est choisi dans le groupe constitué par les composés dans lesquels des groupements époxy sont portés par une chaîne aliphatique, une chaîne aromatique, ou une chaîne hérérocyclique.

32. Procédé selon la revendication 27, **caractérisé en ce qu'**il consiste à mélanger le photoinitiateur avec au moins un monomère ou prépolymère capable de polymériser par voie cationique, et à soumettre le mélange obtenu à un rayonnement actinique, y compris un rayonnement β.

33. Procédé selon la revendication 27, **caractérisé en ce que** le photoinitiateur est utilisé sous forme d'une solution dans un solvant inerte vis à vis de la réaction de polymérisation.

34. Procédé selon la revendication 33, **caractérisé en ce que** le solvant inerte est choisi dans le groupe constitué par l'acétone, la méthyl-éthyl cétone, l'acétonitrile, le carbonate de propylène, la g-butyrolactone, les éther-esters des mono-, di-, tri- éthylène ou propylène glycols, les éther-alcools des mono-, di-, tri- éthylène ou propylène glycols, les esters de l'acide phtalique ou de l'acide citrique.

35. Procédé selon la revendication 27, **caractérisé en ce que** la réaction est effectuée en présence d'un solvant ou d'un diluant constitué par un composé réactif vis à vis de la polymérisation.

36. Procédé selon la revendication 35, **caractérisé en ce que** le composé réactif est choisi dans le groupe constitué par les mono et di éthers vinyliques des mono-, di-, tri-, tétra- éthylène ou propylène glycols, le trivinyl éther triméthylolpropane et le divinyléther du diméthanol-cyclohexane, la N-vinylpyrolidone, le 2-propényléther du carbonate de propylène.

37. Procédé selon la revendication 27, **caractérisé en ce que** l'on ajoute un photosensibilisateur au milieu réactionnel.

38. Procédé selon la revendication 37, **caractérisé en ce que** le photosensibilisateur est choisi dans le groupe constitué par l'anthracène, le diphényl-9,10-anthracène, le pérylène, la phénothiazine, le tétracène, la xanthone, la thioxanthone, l'isopropylthioxantone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles.

39. Procédé selon la revendication 27, **caractérisé en ce que** le mélange réactionnel contient en outre au moins un monomère ou prépolymère capable de polymériser par voie radicalaire et un composé capable de libérer un amorceur de polymérisation radicalaire sous l'effet du rayonnement actinique ou du rayonnement β ou sous l'action de la chaleur.

40. Composition de colorant cationique, **caractérisée en ce qu'**elle contient un composé selon la revendication 1.

41. Composition de colorant cationique selon la revendication 40, **caractérisé en ce que** la ou les charges négatives du groupement anionique R_{D}-Y-C(C≡N)₂⁻ ou Z-C(C≡N)₂⁻ sont soit fixées à la molécule de colorant, soit elles constituent le contre-ion des charges positives du colorant.

42. Utilisation d'un composé selon la revendication 1, comme solvant pour effectuer des réactions chimiques, photochimiques, électrochimiques, photoélectrochimiques, ledit composé étant utilisé au dessus de son point de fusion.

## Claims

1. Ionic compound derived from malononitrile comprising an anionic part associated to at least one cationic part M^{m+} in sufficient number to provide electronic neutrality of the assembly, **characterized in that** the anionic part corresponds to one of the formula R_{D}-Y-C(C≡N)₂⁻ or Z-C(C≡N)₂ in which :
- Z represents an electroattractor radical having a Hammett parameter at least equal to that of a phenyl radical, selected from :
j) -SCN, -N₃, FSO₂-, -CF₃, R'_{F}CH₂- (R'_{F} being a perfluorinated radical, preferably CF₃), fluoroalkyloxy, fluoroalkylthioxy, fluoroalkenyloxy, fluoroalkenylthioxy radicals ;
jj) radicals comprising one or more aromatic nuclei possibly containing at least one nitrogen, oxygen, sulfur or phosphorus atom, said nuclei possibly being condensed nuclei and/or said nuclei possibly carrying at least one substituent selected from halogens, -CN, -NO₂, -SCN, -N₃, CF₂=CF-O- radicals, R_{F}- and R_{F}-CH₂- in which R_{F} represents a perfluoroalkyl radical having 1 to 12 carbon atoms, fluoroalkyloxy groups, fluoroalkylthioxy groups, alkyl, alkenyl, oxaalkyl, oxa-alkenyl, aza-alkyl, aza-alkenyl, thia-alkyl, and thia-alkenyl radicals, polymer radicals, radicals having at least one cationic ionophorous group and/or at least one anionic ionophorous group ;
- Y represents a sulfonyl group, or a phosphonyl group and :
- R_{D} is a radical selected from :
a) alkyl or alkenyl radicals, aryl, arylalkyl, alkylaryl or alkenylaryl radicals, alicyclic or heterocyclic radicals, including polycyclic radicals ;
b) alkyl or alkenyl radicals comprising at least one functional ether, thioether, amine, imine, amide, carboxyl, carbonyl, isocyanate, isothiocyanate, hydroxy ;
c) aryl, arylalkyl, arylalkenyl, alkylaryl or alkenylaryl radicals, in which the aromatic nuclei and/or at least one substituent of the nucleus comprises heteroatoms such as nitrogen, oxygen, sulfur ;
d) radicals comprising condensed aromatic cycles which possibly comprise at least one heteroatom selected from nitrogen, oxygen, sulfur ;
e) halogenated or perhalogenated alkyl, alkenyl, aryl, arylalkyl, alkylaryl radicals, said radicals possibly comprising functional ether, thioether, imine, amine, carboxyl, carbonyl or hydroxy groups ;
f) radicals R_{C}C(R')(R")-O- in which R_{C} is an alkyl perfluorinated radical and R' and R" are independently from one another an hydrogen atom or a radical such as defined in a), b), c) or d) above [for example CF₃CH₂O-, (CF₃)₃CO-, (CF₃)₃CHO-, CF₃CH(C₆H₅)O-, -CH₂(CF₂)₂CH₂-] ;
g) radicals (R_{B})₂N-, in which the radicals R_{B} which are identical or different are such as defined in a), b), c), d) and e) above, one of the R_{B} may be a halogen atom, or the two radicals R_{B} together form a divalent radical which constitutes a cycle with N ;
h) polymer radicals,
i) radicals having one or more cationic ionophorous groups and/or one or more anionic ionophorous groups ;
and M^{m+} is an onium cation selected from the group consisting of R₃O⁺ (oxonium), NR₄⁺ (ammonium), RC(NR₂)₂⁺ (amidinium), C(NR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium), the radicals R representing independently from one another a H or a radical selected from the group consisting of :
- alkyl, alkenyl, oxa-alkyl, oxa-alkenyl, aza-alkyl, aza-alkenyl, thia-alkyl, thia-alkenyl, aryl, arylalkyl, alkylaryl, alkenylaryl radicals, dialkylamino radicals and dialkylazo radicals ;
- cyclic or heterocyclic radicals optionally comprising at least one side chain comprising heteroatoms such as nitrogen, oxygen , sulfur ;
- cyclic or heterocyclic radicals optionally comprising heteroatoms in the aromatic nucleus ;
- groups comprising a plurality of aromatic or heterocyclic nuclei, condensed or non-condensed, optionally containing at least one nitrogen, oxygen, sulfur or phosphorus atom ;with the proviso that:
• compounds C₆H₅SO₂C(CN)₂NH₄, C₆H₅SO₂C(CN)₂C₆H₅CH₂-SC(NH₂)NH, C₆H₅SO₂C(CN)₂C₆H₅NH₃, m-NO₂C₆H₅SO₂C(CN)₂(Et)₃NH, C₆H₅SO₂C(CN)₂C₅H₁₀NH et C₆H₅SO₂C(CN)₂C₅H₅NH are excluded ;
• substituent Z may be a monovalent radical, part of radical having a valence greater than 2 and carrying a plurality of -Y-C⁻(C≡N)₂ groups, or a segment of a polymer ;
• substituent R_{D} may be a monovalent radical, part of radical having a valence greater than 2 and carrying a plurality of -Y-C⁻(C≡N)₂ groups, or a segment of a polymer ;
• "segment of a polymer" means an ethylene repeat unit optionally having a alkylene substituent or an oxyalkenylene substituent, an oxyalkylene repeat unit optionally having a methylene group (derived form 2,3-epoxymethane), a styrene repeat unit optionally having a polyoxyethylene substituent, an azaethylene repeat unit, a 2-(triethoxysilyl)ethyl-co-O-[2-(trimethoxysilyl)ethyl]-O'-methylpolyethylene glycol repeat unit, a bis[3-trimethoxysilyl)propyl]amino repeat unit, a methylethylsiloxane repeat unit, an acrylonitrile-co-4-styrenyl repeat unit, or an aniline unit having a fluoroalkyl substituent ;
• when a cation has at least two R substituents different from H, said substituents may form together aromatic rings or non aromatic rings optionally enclosing the center bearing the cationic charge ;
• "cationic ionophorous group" means an iodonium, sulfonium, oxonium, ammonium, amidinium, guanidinium, pyridinium, imidazolium, imidazolinium, triazolium, phosphonium or carbonium group, said ionic group acting totally or partly as the cation M⁺ ;
• "anionic ionophoric group" means a carboxylate function (-CO₂⁻), a sulfonate function (-SO₃⁻), a sulfonimide function (-SO₂NSO₂-) or a sulfonamide function (-SO₂N-).

2. Compound according to claim 1, **characterized in that** the onium cation is part of the radical Z or the radical R_{D}.

3. Compound according to claim 1, **characterized in that** the onium cation is part of a recurring unit of a polymer.

4. Compound according to claim 1, **characterized in that** the cation M⁺ is a cationic heterocycle with aromatic character, including at least one nitrogen atom which is alkylated in the cycle.

5. Compound according to claim 4, **characterized in that** the cation is an imidazolium, a triazolium, a pyridinium, a 4-dimetyl-amino-pyridinium, said cations possibly carrying a substituent on the carbon atoms of the cycle.

6. Compound according to claim 1, **characterized in that** the cation M is a group having a bond -N=N, -N=N⁺, a sulfonium group, an iodonium group, or an arene-ferrocenium cation, which is substituted or non-substituted, possibly incorporated in a polymeric network.

7. Compound according to claim 2, **characterized in that** the cation is a diaryliodonium cation, a dialkylaryliodonium cation, a triarylsulfonium cation, a trialkylaryl sulfonium cation, or a substituted or non-substituted phenacyl-dialkyl sulfonium cation.

8. Compound according to claim 1, **characterized in that** the aryliodonium cation, or the alkylaryliodonium cation, or the triarylsulfonium cation, or the alkylaryl sulfonium cation, or the phenacyl-dialkyl sulfonium cation are part of a polymer chain.

9. Compound according to claim 1, **characterized in that** M is an organic cation incorporating a group 2,2'[Azobis(2-2'-imidazolinio-2-yl)propane]²⁺ or 2,2'-Azobis(2-amidiniopropane)²⁺.

10. Compounds according to claim 1, **characterized in that** the substituent Z is selected from the group consisting of -OCₙF₂ₙ₊₁, -OC₂F₄H, -SCₙF₂ₙ₊₁ and -SC₂F₄H, -O-CF=CF₂, -SCF=CF₂, n being a whole number from 1 to 8.

11. Compound according to claim 1, **characterized in that** Z is a radical CₙF₂ₙ₊₁CH₂-, n being a whole number from 1 to 8.

12. Compound according to claim 1, **characterized in that** R_{D} is a radical selected from alkyl, alkenyl, oxa-alkyl, oxa-alkenyl, aza-alkyl, aza-alkenyl, thia-alkyl or thia-alkenyl having 1 to 24 carbon atoms, or from aryl, arylalkyl, alkylaryl or alkenylaryl having 5 to 24 carbon atoms.

13. Compound according to claim 1, **characterized in that** R_{D} is a radical selected from alkyl or alkenyl radicals having 1 to 12 carbon atoms and possibly comprising at least one heteroatom O, N or S in the main chain or in a lateral chain, and/or possibly carrying a hydroxy group, a carbonyl group, an amino group, or a carboxyl group.

14. Compound according to claim 1, **characterized in that** R_{D} is a radical selected from aryl, arylalkyl, alkylaryl or alkenylaryl, in which the aromatic nuclei and/or their substituents comprise heteroatoms such as nitrogen, oxygen, sulfur.

15. Compound according to claim 1, **characterized in that** R_{D} is part of a poly (oxyalkylene) radical or a polystyrene radical.

16. Compound according to claim 1, **characterized in that** R_{D} is a radical having an iodonium, sulfonium, oxonium, ammonium, amidinium, guanidinium, pyridinium, imidazolium, triazolium, phosphonium or carbonium, said ionic group behaving totally or partially as cation M⁺.

17. Compound according to claim 1, **characterized in that** R_{D} has one or more anionic ionophorous groups consisting of a carboxylic function (-CO₂⁻), a sulfonic function (-SO₃⁻), a sulfonimide function (-SO₂NSO₂-) or a sulfonamide function (-SO₂N-).

18. Compound according to claim 1, **characterized in that** R_{D} includes at least one ethylenic unsaturation and/or a condensable group and/or a group which is dissociable by thermal means, by photochemical means or by ionic dissociation.

19. Compound according to claim 1, **characterized in that** R_{D} represents a mesomorphous group or a chromophore group or a self-doped electronically conductive polymer or a hydrolysable alkoxysilane.

20. Compound according to claim 1, **characterized in that** R_{D} represents a recurring unit or a polymer chain.

21. Compound according to claim 1, **characterized in that** R_{D} includes a group capable of trapping free radicals.

22. Compound according to claim 1, **characterized in that** R_{D} includes a dissociating dipole.

23. Compound according to claim 1, **characterized in that** R_{D} includes a redox couple.

24. Compound according to claim 1, **characterized in that** R_{D} includes a complexing ligand.

25. Compound according to claim 1, **characterized in that** R_{D}-Y- is optically active.

26. Compound according to claim 1, **characterized in that** R_{D} represents a radical having a valency v higher than 2, including at both free ends a group -C(C≡N)₂⁻.

27. A method for polymerization or crosslinking of monomers or prepolymers capable of reacting cationically, **characterized in that** a compound according to claim 1 is used as a photoinitiateur source of acid catalyzing the reaction.

28. A method according to claim 27, **characterized in that** the ionic compound is selected from the compounds wherein the cation has a bond -N=N-, -N=N⁺, a sulfonium group, an iodonium group, or an arene-ferrocenium cation, which is substituted or non-substituted, possibly incorporated in a polymeric network

29. A method according to claim 27, **characterized in that** the R_{D} substituent of the ionic compound is a non substituted alkyl group, a group comprising an oxa group or a sulfone, a group comprising a sulfoxide, sulfone, phosphine oxide or phosphonate group.

30. A method according to claim 27, **characterized in that** the monomers are selected from the group consisting of compounds which include a cyclic ether function, a cyclic thioether function or a cyclic amine function, vinyl compounds, vinyl ether compounds, oxazolines, lactones and lactames.

31. A method according to claim 27, **characterized in that** the prepolymer is selected from the group consisting of the compounds having epoxy groups on an aliphatic chain, an aromatic chain, or a heterocyclic chain.

32. A method according to claim 27, **characterized in that** it consists in mixing the photoinitiator with at least one monomer or prepolymer capable of cationic polymerization, and subjecting the mixture obtained to actinic radication, including β radiation.

33. A method according to claim 33, **characterized in that** the photoinitiator is used in the form of a solution in an solvent which is inert to the polymerization reaction.

34. A method according to claim 27, **characterized in that** the inert solvent is selected from the group consisting of acetone, methyl-ethyl cetone, acetonitrile, propylene carbonate, γ-butyrolactone, ether-esters of mono-, di-, tri-ethylene or propylene glycols, ether-alcohols of mono-, di-, tri-ethylene or propylene glycols, esters of phtalic acid or of citric acid.

35. A method according to claim 27, **characterized in that** the reaction is carried on in the presence of a solvent or a diluent consisting of a compound which is reactive towards polymerization.

36. A method according to claim 35, **characterized in that** the reactive compound is selected from the group consisting of mono-, di-, tri-, tetra-ethylene or propylene glycols, trimethylolpropane trivinylether, dimethanol-cyclohexane divinylether N-vinylpyrolidone, and 2-propenylether of propylene carbonate.

37. A method according to claim 27, **characterized in that** a photosensitizer is added to the reaction medium.

38. A method according to claim 27, **characterized in that** the photosensitizer is selected from the group consisting of anthracene, diphenyl-9,10-anthracene, perylene, phenothiazine, tetracene, xanthone, thioxanthone, isopropylthioxantone, acetophenone, benzophenone, 1,3,5-triaryl-2-pyrazolines and derivatives thereof, particularly derivatives which are substituted on the aromatic nuclei by alkyl, oxa- or aza-alkyl radicals.

39. A method according to claim 27, **characterized in that** the reaction mixture further contains at least one monomer or prepolymer capable of polymerizing by free radical and a compound which is capable to produce a free radical initiator thermally or by action of actinic radiation of β-radiation.

40. Cationic coloring composition, **characterized in that** it contains a compound according to claim 1.

41. A cationic coloring composition according to claim 40, **characterized in that** the negative charge(s) of the anionic group R_{D}-Y-C(C≡N)₂⁻ or Z-C(C≡N)₂ are either fixed to the coloring molecule, or they constitute the counter-ion of the positive charges of the coloring molecule.

42. Use of a compound according to claim 1, as the solvent to carry out chemical, photochemical, photoelectrochemical reactions, said compound being used at a tempeture higher than the melting point.

## Patentansprüche

1. Von Malononitril abgeleitete ionische Verbindung, die einen anionischen Anteil umfasst, der mit zumindest einem kationischen Anteil M^{m+} in ausreichender Anzahl assoziiert ist, um insgesamt elektrische Neutralität zu gewährleisten, **dadurch gekennzeichnet, dass** der anionische Anteil einer der Formeln R_{D}-Y-C(C≡N)₂⁻ oder Z-C(C≡N)₂⁻ entspricht, worin:
- Z einen Elektronen anziehenden Rest mit einem Hammett-Parameter darstellt, der zumindest dem eines Phenylrests entspricht, ausgewählt aus:
j) -SCN, -N₃, -CF₃, FSO₂-, R'_{F}CH₂- (wobei R'_{F} ein perfluorierter Rest ist), Fluoralkyloxy-, Fluoralkylthio-, Fluoralkenyloxy- und Fluoralkenylthio-Resten;
jj) Resten mit einem oder mehreren aromatischen Kernen, die gegebenenfalls zumindest ein Stickstoff-, Sauerstoff-, Schwefel- oder Phosphoratom enthalten, wobei die Kerne gegebenenfalls kondensierte Kerne sind und/oder die Kerne gegebenenfalls zumindest einen Substituenten aufweisen, der aus Halogenen, -CN, -NO₂, -SCN, -N₃, CF₂=CF-O-, den Resten R_{F}- und R_{F}-CH₂-, worin R_{F} für einen Perfluoralkylrest mit 1 bis 12 Kohlenstoffatomen steht, Fluoralkyloxygruppen, Fluoralkylthiogruppen, Alkyl-, Alkenyl, Oxaalkyl-, Oxaalkenyl-, Azaalkyl-, Azaalkenyl-, Thiaalkyl-, Thiaalkenylresten, Polymerresten, sowie Resten, die zumindest eine kationische Ionophorgruppe und/oder zumindest eine anionische Ionophorgruppe aufweisen, ausgewählt ist;
- Y für eine Sulfonylgruppe oder eine Phosphonylgruppe steht; und
- R_{D} ein aus Folgenden ausgewählter Rest ist:
a) Alkyl- oder Alkenylresten, Aryl-, Arylalkyl-, Alkylaryl- oder Alkenylarylresten, alizyklischen oder heterozyklischen Resten, einschließlich polyzyklischer Reste;
b) Alkyl- oder Alkenylresten, die zumindest eine Ether-, Thioether-, Amin-, Imin-, Amid-, Carboxyl-, Carbonyl-, Isocyanat-, lsothiocyanat-, Thiocarbonyl- oder Hydroxy-Funktionalität umfassen;
c) Aryl-, Arylalkyl-, Arylalkenyl-, Alkylaryl- oder Alkenylarylresten, worin die aromatischen Kerne und/oder zumindest ein Kernsubstituent Heteroatome, wie z.B. Stickstoff, Sauerstoff oder Schwefel, umfassen;
d) Resten, die kondensierte aromatische Ringe umfassen, die gegebenenfalls zumindest ein aus Stickstoff, Sauerstoff und Schwefel ausgewähltes Heteroatom umfassen;
e) halogenierten oder perhalogenierten Alkyl-, Alkenyl-, Aryl-, Arylalkyl- oder Alkylarylresten, wobei die Reste gegebenenfalls Ether-, Thioether-, Imin-, Amin-, Carboxyl-, Carbonyl oder Hydroxy-Funktionalitäten umfassen;
f) Resten R_{C}C(R')(R")-O-, worin R_{C} ein perfluorierter Alkylrest ist und R' und R" jeweils unabhängig voneinander ein Wasserstoffatom oder ein Rest wie zuvor unter a), b), c) oder d) definiert sind;
g) Resten (R_{B})₂N-, worin die Reste R_{B}, die identisch oder unterschiedlich sein können, wie zuvor unter a), b), c), d) oder e) definiert sind, wobei ein R_{B} ein Wasserstoffatom sein kann, oder worin die beiden Reste R_{B} miteinander einen zweiwertigen Rest bilden, der mit N einen Ring bildet;
h) Polymerresten;
i) Resten, die eine oder mehrere kationische Ionophorgruppen und/oder eine oder mehrere anionische lonophorgruppen aufweisen;
und **dadurch gekennzeichnet, dass** das Kation M^{m+} ein Oniumkation ist, das aus der aus den Kationen R₃O⁺ (Oxonium), NR₄⁺ (Ammonium), RC(NR₂)₂⁺ (Amidinium), C(NR₂)₃⁺ (Guanidinium), C₅R₆N⁺ (Pyridinium), C₃R₅N₂⁺ (Imidazolium), C₃R₇N₂⁺ (Imidazolinium), C₂R₄N₃⁺ (Triazolium), SR₃⁺ (Sulfonium), PR₄⁺ (Phosphonium), IR₂⁺ (Iodonium) und (C₆R₅)₃C⁺ (Carbonium) bestehenden Gruppe ausgewählt ist, wobei die Reste R jeweils unabhängig voneinander ein H oder einen Rest darstellen, der aus der aus Folgenden bestehenden Gruppe ausgewählt ist:
- Alkyl-, Alkenyl-, Oxaalkyl-, Oxaalkenyl-, Azaalkyl-, Azaalkenyl-, Thiaalkyl-, Thiaalkenyl-, Aryl-, Arylalkyl-, Alkylaryl-, Alkenylarylresten, Dialkylaminoresten und Dialkylazoresten;
- zyklischen oder heterozyklischen Resten, die gegebenenfalls zumindest eine Seitenkette umfassen, die Heteroatome, wie z.B. Stickstoff, Sauerstoff oder Schwefel, umfasst;
- zyklischen oder heterozyklischen Resten, die gegebenenfalls Heteroatome im aromatischen Kern umfassen;
- Gruppen, die mehrere kondensierte oder nichtkondensierte, aromatische oder heterozyklische Kerne umfassen, die gegebenenfalls zumindest ein Stickstoff-, Sauerstoff-, Schwefel- oder Phosphoratom enthalten;
mit der Maßgabe, dass:
• die Verbindungen C₆H₅SO₂(CN)₂NH₄, C₆H₅SO₂C(CN)₂C₆H₅CH₂-SC(NH₂)NH, C₆H₅SO₂C(CN)₂C₆H₅NH₃, m-NO₂C₆H₅SO₂C(CN)₂(Et)₃NH, C₆H₅SO₂C(CN)₂₋C₅H₁₀NH und C₆H₅SO₂C(CN)₂C₅H₅NH ausgenommen sind;
• ein Substituent Z ein einwertiger Rest, ein Teil eines Rests mit einer Wertigkeit von 2, der zwei Gruppen -C⁻(C≡N)₂ trägt, oder ein Segment eines Polymers sein kann;
• ein Substituent R_{D} ein einwertiger Rest, ein Teil eines Rests mit einer Wertigkeit von über 2, der mehrere Gruppen -Y-C⁻(C≡N)₂ trägt, oder ein Segment eines Polymers sein kann;
• "Segment eines Polymers" für eine Ethylen-Grundeinheit, die gegebenenfalls einen Alkylensubstituenten oder einen Oxyalkenylensubstituenten aufweist, eine Oxyalkylen-Grundeinheit, die gegebenenfalls eine Methylengruppe (abgeleitet von 2,3-Epoxypropan) aufweist, eine Styrol-Grundeinheit, die gegebenenfalls einen Polyoxyethylensubstituenten aufweist, eine Azaethylen-Grundeinheit, eine 2-(Triethoxysilyl)ethyl-co-O-[2-(trimethoxysilyl)ethyl]-O'-methylpolyethylenglykol-Grundeinheit, eine Bis[(3-trimethoxysilyl)propyl]amino-Grundeinheit, eine Methylethylsiloxan-Grundeinheit, eine Acrylnitril-co-4-styrenyl-Grundeinheit oder eine Anilin-Grundeinheit, die einen Fluoralkylsubstituenten aufweist, steht;
• wenn ein Kation zumindest zwei Reste R aufweist, die nicht H sind, diese Reste miteinander aromatische Ringe bilden können, die gegebenenfalls das die kationische Ladung tragende Zentrum umfassen;
• die "kationische Ionophorgruppe" eine Iodonium-, Sulfonium- Oxonium-, Ammonium-, Amidinium-, Guanidinium-, Pyridinium- Imidazolium-, Imidazolinium, Triazolium-, Phosphonium- oder Carboniumgruppe bezeichnet, wobei diese ionischen Gruppen vollständig oder teilweise die Rolle des Kations M⁺ übernehmen;
• die "anionische Ionophorgruppe" eine Carboxylat-Funktionalität (-CO₂⁻), eine Sulfonat-Funktionalität (-SO₃⁻), eine Sulfonimid-Funktionalität (-SO₂NSO₂⁻) oder eine Sulfonamid-Funktionalität (-SO₂N-) bezeichnet.

2. Verbindung nach Anspruch 1, **dadurch gekennzeichnet, dass** das Oniumkation Bestandteil des Rests Z oder des Rests R_{D} ist.

3. Verbindung nach Anspruch 1, **dadurch gekennzeichnet, dass** das Oniumkation Bestandteil einer Grundeinheit eines Polymers ist.

4. Verbindung nach Anspruch 1, **dadurch gekennzeichnet, dass** das Kation M⁺ ein aromatischer kationischer Heterozyklus ist, der im Ring zumindest ein alkyliertes Stickstoffatom aufweist.

5. Verbindung nach Anspruch 4, **dadurch gekennzeichnet, dass** das Kation Imidazolium, Imidazolinium, Triazolium, Pyridinium oder 4-Dimethylaminopyridinium ist, wobei diese Kationen gegebenenfalls einen Substituenten auf den Kohlenstoffatomen des Rings tragen.

6. Verbindung nach Anspruch 1, **dadurch gekennzeichnet, dass** das Kation M eine Gruppe ist, die eine Bindung -N=N oder -N=N⁺, eine Sulfoniumgruppe, eine Iodoniumgruppe oder ein substituiertes oder unsubstituiertes Aren-Ferrocen-Kation aufweist und gegebenenfalls in einem Polymer inkorporiert ist.

7. Verbindung nach Anspruch 1, **dadurch gekennzeichnet, dass** das Kation ein substituiertes oder unsubstituiertes Diaryliodoniumkation, Dialkylaryliodoniumkation, Triarylsulfoniumkation, Trialkylarylsulfoniumkation oder Phenacyldialkylsulfoniumkation ist.

8. Verfahren nach Anspruch 1, **dadurch gekennzeichnet, dass** das Aryliodoniumkation, das Alkylaryliodoniumkation, das Triarylsulfoniumkation, das Alkylarylsulfoniumkation oder das Phenacyldialkylsulfoniumkation Teil einer Polymerkette ist.

9. Verbindung nach Anspruch 1, **dadurch gekennzeichnet, dass** M ein organisches Kation ist, das eine Gruppe 2,2'-[Azobis(2,2'-imidazolinio-2-yl)propan]²⁺ oder 2,2'-Azobis(2-amidiniopropan)²⁺ umfasst.

10. Verbindung nach Anspruch 1, **dadurch gekennzeichnet, dass** Z aus der aus -OCₙF₂ₙ₊₁, -OC₂F₄H, -SCₙF₂ₙ₊₁ und -SC₂F₄H, -OCF=CF₂, -SCF=CF₂ bestehenden Gruppe ausgewählt ist, worin n eine Zahl von 1 bis 8 ist.

11. Verbindung nach Anspruch 1, **dadurch gekennzeichnet, dass** Z ein Rest CₙF₂ₙ₊₁CH₂- ist, worin n eine ganze Zahl von 1 bis 8 ist.

12. Verbindung nach Anspruch 1, **dadurch gekennzeichnet, dass** R_{D} aus Alkyl-, Alkenyl-, Oxaalkyl-, Oxaalkenyl-, Azaalkyl-, Azaalkenyl-, Thiaalkyl- oder Thiaalkenylresten mit 1 bis 24 Kohlenstoffatomen ausgewählt ist oder aus Aryl-, Arylalkyl-, Alkylaryl- oder Alkenylarylresten mit 5 bis 24 Kohlenstoffatomen ausgewählt ist.

13. Verbindung nach Anspruch 1, **dadurch gekennzeichnet, dass** R_{D} aus Alkyl- oder Alkenylresten mit 1 bis 12 Kohlenstoffatomen ausgewählt ist, die gegebenenfalls zumindest ein Heteroatom O, N oder S in der Hauptkette oder in einer Seitenkette umfassen und/oder die gegebenenfalls eine Hydroxygruppe, eine Carbonylgruppe, eine Aminogruppe oder eine Carboxylgruppe aufweisen.

14. Verbindung nach Anspruch 1, **dadurch gekennzeichnet, dass** R_{D} aus Aryl-, Arylalkyl-, Alkylaryl- oder Alkenylarylresten ausgewählt ist, worin die aromatischen Kerne und/oder ihre Substituenten Heteroatome, wie z.B. Stickstoff, Sauerstoff oder Schwefel, umfassen.

15. Verbindung nach Anspruch 1, **dadurch gekennzeichnet, dass** R_{D} Teil eines Poly(oxyalkylen)rests oder eines Polystyrolrests ist.

16. Verbindung nach Anspruch 1, **dadurch gekennzeichnet, dass** R_{D} ein Rest ist, der eine Iodoniumgruppe, eine Sulfonium-, Oxonium-, Ammonium-, Amidinium-, Guanidinium-, Pyridinium-, Imidazolium-, lmidazolinium-, Triazolium-, Phosphonium- oder Carboniumgruppe aufweist, wobei die ionische Gruppe vollständig oder teilweise die Rolle des Kations M⁺ übernimmt.

17. Verbindung nach Anspruch 1, **dadurch gekennzeichnet, dass** R_{D} eine oder mehrere anionische lonophorgruppen aufweist, die durch eine Carboxylatfunktionalität (-CO₂⁻), eine Sulfonatfunktionalität (-SO₃⁻), eine Sulfonimidfunktionalität (-SO₂NSO₂-) oder eine Sulfonamidfunktionalität (-SO₂N-) gebildet werden.

18. Verbindung nach Anspruch 1, **dadurch gekennzeichnet, dass** R_{D} zumindest eine ethylenische Unsättigung und/oder eine kondensierbare Gruppe und/oder eine thermisch, photochemisch oder durch ionische Dissoziation dissoziierbare Gruppe umfasst.

19. Verbindung nach Anspruch 1, **dadurch gekennzeichnet, dass** R_{D} eine mesomorphe Gruppe oder eine chromophore Gruppe oder ein eigendotiertes elektronenleitendes Polymer oder ein hydrolysierbares Alkoxysilan darstellt.

20. Verbindung nach Anspruch 1, **dadurch gekennzeichnet, dass** R_{D} eine Grundeinheit einer Polymerkette darstellt.

21. Verbindung nach Anspruch 1, **dadurch gekennzeichnet, dass** R_{D} eine Gruppe trägt, die fähig ist, freie Radikale einzufangen.

22. Verbindung nach Anspruch 1, **dadurch gekennzeichnet, dass** R_{D} einen dissoziierenden Dipol trägt.

23. Verbindung nach Anspruch 1, **dadurch gekennzeichnet, dass** R_{D} ein Redoxpaar trägt.

24. Verbindung nach Anspruch 1, **dadurch gekennzeichnet, dass** R_{D} einen komplexbildenden Liganden trägt.

25. Verbindung nach Anspruch 1, **dadurch gekennzeichnet, dass** R_{D} optisch aktiv ist.

26. Verbindung nach Anspruch 1, **dadurch gekennzeichnet, dass** R_{D} einen Rest mit einer Wertigkeit v von über 2 darstellt, der an jedem freien Ende eine Gruppe -C(C≡N)₂⁻ aufweist.

27. Verfahren zur Polymerisation oder Vernetzung von Monomeren oder Präpolymeren, die in der Lage sind, kationisch zu reagieren, **dadurch gekennzeichnet, dass** eine Verbindung nach Anspruch 1 als Photoinitiator-Säurequelle verwendet wird, die die Reaktion katalysiert.

28. Verfahren nach Anspruch 27, **dadurch gekennzeichnet, dass** die ionische Verbindung aus jenen ausgewählt ist, in denen das Kation eine Bindung -N=N⁺, -N=N-, eine Sulfoniumgruppe, eine Iodoniumgruppe oder ein substituiertes oder unsubstituiertes Aren-Ferrocen-Kation umfasst und gegebenenfalls in einem Polymer inkorporiert ist.

29. Verfahren nach Anspruch 27, **dadurch gekennzeichnet, dass** der Substituent R_{D} der ionischen Verbindung ein unsubstituierter Alkylrest, ein eine Oxa- oder Sulfongruppe enthaltender Rest oder ein eine Sulfoxid-, Sulfon-, Phosphinoxid- oder Phosphonatgruppe enthaltender Rest ist.

30. Verfahren nach Anspruch 27, **dadurch gekennzeichnet, dass** die Monomere aus der aus Verbindungen, die eine zyklische Etherfunktionalität, eine zyklische Thioetherfunktionalität oder eine zyklische Aminfunktionalität aufweisen, Vinylverbindungen, Vinylethern, Oxazolinen, Lactonen und Lactamen bestehenden Gruppe ausgewählt sind.

31. Verfahren nach Anspruch 27, **dadurch gekennzeichnet, dass** das Präpolymer aus der aus Verbindungen, in denen Epoxygruppen von einer aliphatischen Kette, einer aromatischen Kette oder einer heterozyklischen Kette getragen werden, bestehenden Gruppe ausgewählt ist.

32. Verfahren nach Anspruch 27, **dadurch gekennzeichnet, dass** es darin besteht, den Photoinitiator mit zumindest einem Monomer oder Präpolymer zu vermischen, das in der Lage ist, kationisch zu polymerisieren, und das erhaltene Gemisch aktinischer Strahlung, einschließlich β-Strahlung, auszusetzen.

33. Verfahren nach Anspruch 27, **dadurch gekennzeichnet, dass** der Photoinitiator in Form einer Lösung in einem Lösungsmittel eingesetzt wird, das gegenüber der Polymerisationsreaktion inert ist.

34. Verfahren nach Anspruch 33, **dadurch gekennzeichnet, dass** das inerte Lösungsmittel aus der aus Aceton, Methylethylketon, Acetonitril, Propylencarbonat, γ-Butyrolacton, Etherestern von Mono-, Di-, Triethylen- und -propylenglykolen, Etheralkoholen von Mono-, Di-, Triethylen- oder -propylenglykolen, Phthalsäure- und Citronensäurestern bestehenden Gruppe ausgewählt ist.

35. Verfahren nach Anspruch 27, **dadurch gekennzeichnet, dass** die Reaktion in Gegenwart eines Lösungsmittels oder eines Verdünnungsmittels durchgeführt wird, das aus einer gegenüber der Polymerisation reaktiven Verbindung besteht.

36. Verfahren nach Anspruch 35, **dadurch gekennzeichnet, dass** die reaktive Verbindung aus der aus Mono- und Divinylethern von Mono-, Di-, Tri-, Tetraethylen- und -propylenglykolen, Trimethylolpropantrivinylether, Dimethanolcyclohexandivinylether, N-Vinylpyrrolidon und Propylencarbonat-2-propenylether bestehenden Gruppe ausgewählt ist.

37. Verfahren nach Anspruch 27, **dadurch gekennzeichnet, dass** dem Reaktionsmedium ein Photosensibilisator zugesetzt wird.

38. Verfahren nach Anspruch 37, **dadurch gekennzeichnet, dass** der Photosensibilisator aus der aus Anthracen, Diphenyl-9,10-anthracen, Perylen, Phenothiazin, Tetracen, Xanthon, Thioxanthon, Isopropylthioxanthon, Acetophenon, Benzophenon, 1,3,5-Triaryl-2-pyrazolinen und Derivaten davon, insbesondere Derivaten mit Substitutionen an den aromatischen Kernen in Form von Alkyl-, Oxaalkyl- oder Azaalkylresten, bestehenden Gruppe ausgewählt ist.

39. Verfahren nach Anspruch 27, **dadurch gekennzeichnet, dass** das Reaktionsgemisch weiters zumindest ein Monomer oder Präpolymer, das in der Lage ist, radikalisch zu polymerisieren, und eine Verbindung enthält, die in der Lage ist, unter Einwirkung von aktinischer Strahlung oder β-Strahlung oder unter Wärmeeinwirkung einen radikalischen Polymerisationsinitiator freizusetzen.

40. Kationische Farbstoffzusammensetzung, **dadurch gekennzeichnet, dass** sie eine Verbindung nach Anspruch 1 enthält.

41. Kationische Farbstoffzusammensetzung nach Anspruch 40, **dadurch gekennzeichnet, dass** die negative(n) Ladung(en) der anionischen Gruppe R_{D}-Y-C(C≡N)₂⁻ oder Z-C(C≡N)₂⁻ an das Farbstoffmolekül gebunden ist/sind, sodass sie das Gegenion zur positiven Ladung des Farbstoffs bildet/bilden.

42. Verwendung einer Verbindung nach Anspruch 1 als Lösungsmittel zur Durchführung von chemischen, photochemischen, elektrochemischen, photoelektrochemischen Reaktionen, wobei die Verbindung unterhalb ihres Schmelzpunkts eingesetzt wird.
